# EUROPEAN PATENT APPLICATION

(11) **EP 4 748 327 A1**
(43) Date of publication of application: **27.05.2026**
(21) Application number: 24894126.2
(22) Date of filing: 18.11.2024
(51) Int. Cl.: A61B 17/435

(54) **EMBRYO TRANSFER DEVICE KIT**

(30) Priority: 21.11.2023 JP 2023197425
(71) Applicant: Institute of Science Tokyo, Tokyo 152-8550 (JP)
(72) Inventor: FUKUOKA, Muneaki, Tokyo 101-0062 (JP); IKEUCHI, Masashi, Tokyo 152-8550 (JP)
(74) Representative: Nederlandsch Octrooibureau
(86) International application number: PCT/JP2024/040889
(87) International publication number: WO 2025/110135

(57) **Abstract**

An embryo transfer device kit including: a connection component (2) connected to a proximal end of a tube having a distal end to be disposed inside a uterus and the proximal end to be disposed outside the uterus, and an embryo transfer device (1) at least one part of which is accommodated in an accommodation section (71A) provided in the connection component (2) so as to extend in a predetermined direction, wherein the embryo transfer device (1) comprises an embryo accommodation portion (20) having a recess (21) opening in a direction crossing the predetermined direction, and wherein the embryo accommodation portion (20) is for keeping an embryo, which is disposed in the recess (21), in a space surrounded by the recess (21) and an endometrium of a uterine cavity existing in an opening direction of the recess (21), in a state in which the embryo accommodation portion is disposed in the uterine cavity.

## Description

### {Technical Field}

The present disclosure relates to embryo transfer device kits.

### {Background Art}

Conventionally, as an embryo transfer device kit for transferring an embryo into a uterine cavity, one having a sheath whose distal end is disposed in the uterus and whose proximal end is disposed outside the uterus, and a soft tube to be inserted into the sheath, is known. The soft tube is inserted into the sheath from the proximal end side of the sheath so as to protrude from the distal end thereof, and sends the embryo to the uterus via a hollow section thereof. For example, see PTL 1.

### {Citation List}

### {Patent Literature}

{PTL 1} Japanese Unexamined Patent Application Publication No. 2023-126548

### {Summary of Invention}

### {Technical Problem}

Various conditions contribute to improving the probability of implantation, including the force applied from the device to the uterus, damage to the uterine cervix, the uterine cavity, etc. at the time of insertion/withdrawal of the device, and the status of indwelling of the embryo for the purpose of implantation. A practical and effective embryo transfer device kit capable of increasing the probability of implantation is desired.

### {Solution to Problem}

A first aspect is an embryo transfer device kit including: a connection component connected to a proximal end of a tube having a distal end to be disposed inside a uterus and the proximal end to be disposed outside the uterus; and an embryo transfer device at least one part of which is accommodated in an accommodation section provided in the connection component so as to extend in a predetermined direction, wherein the embryo transfer device comprises an embryo accommodation portion having a recess opening in a direction crossing the predetermined direction, and wherein the embryo accommodation portion is for keeping an embryo, which is disposed in the recess, in a space surrounded by the recess and an endometrium of a uterine cavity existing in an opening direction of the recess, in a state in which the embryo accommodation portion is disposed in the uterine cavity.

### {Brief Description of Drawings}

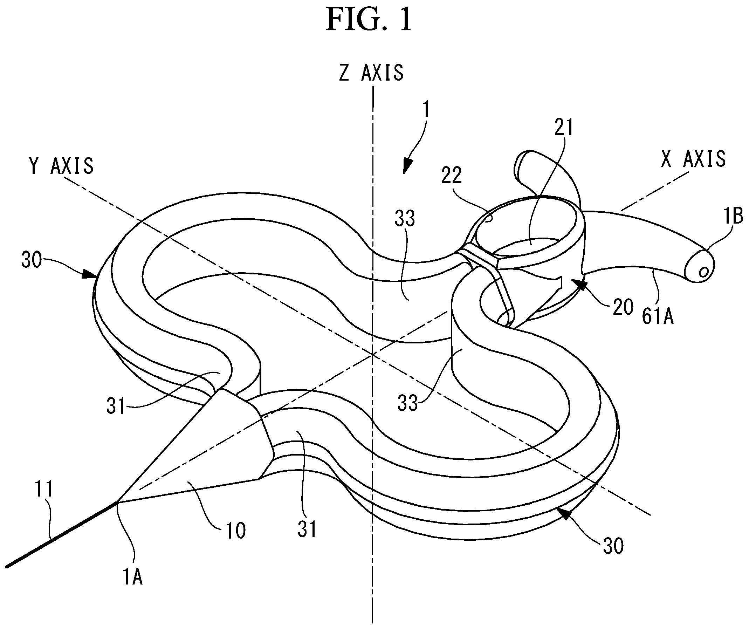

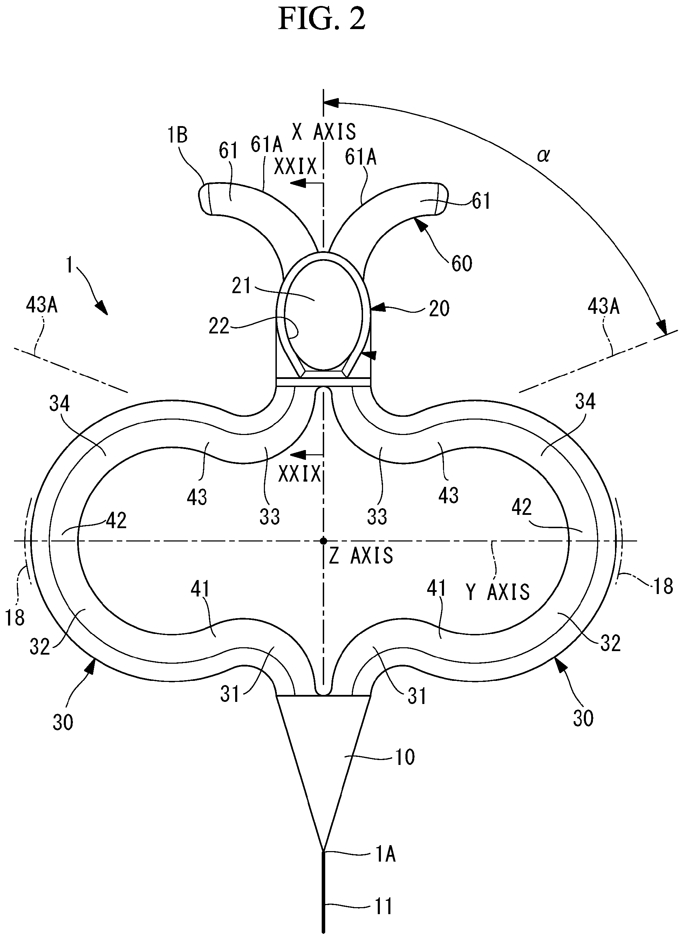

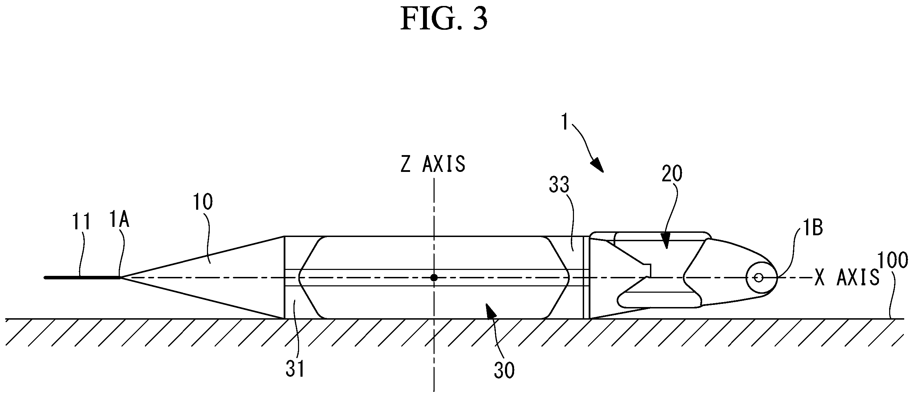

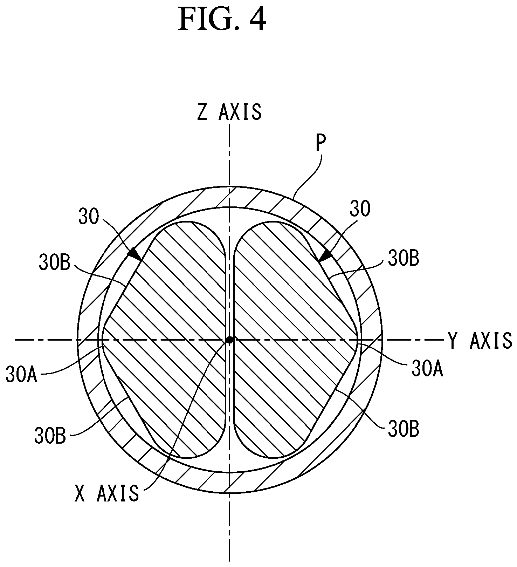

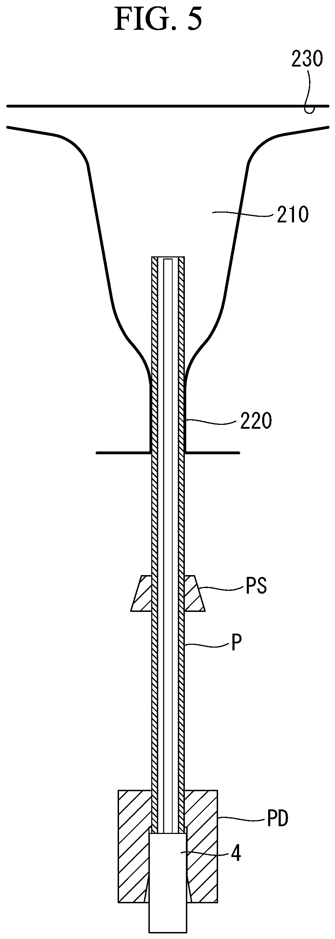

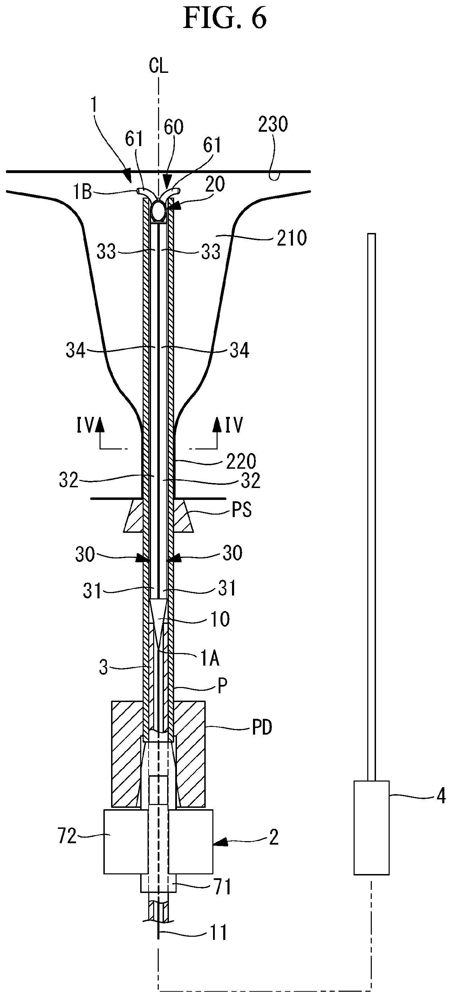

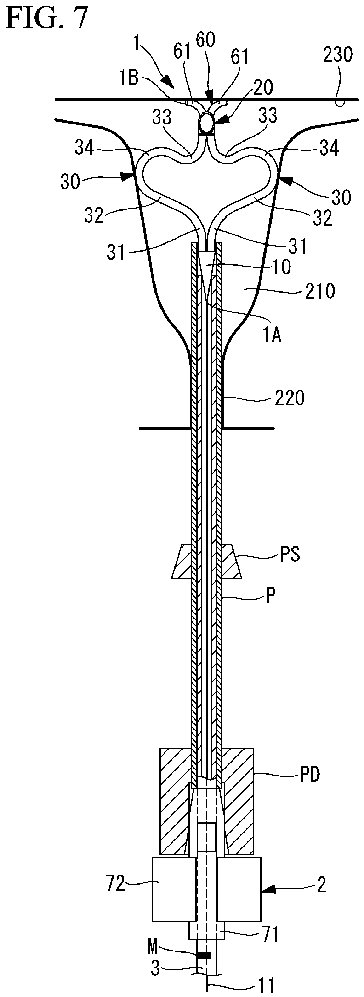

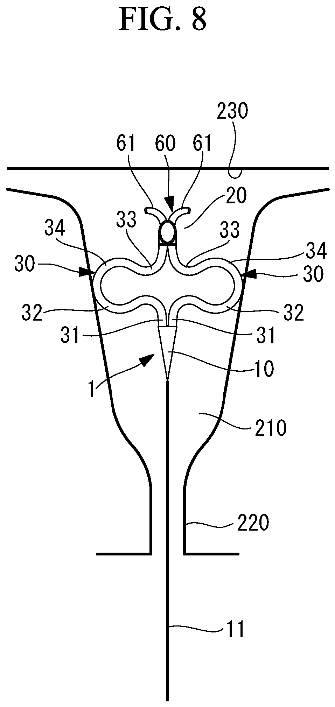

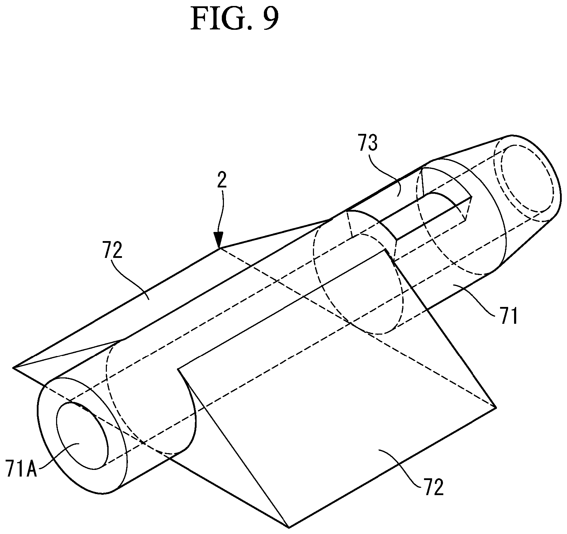

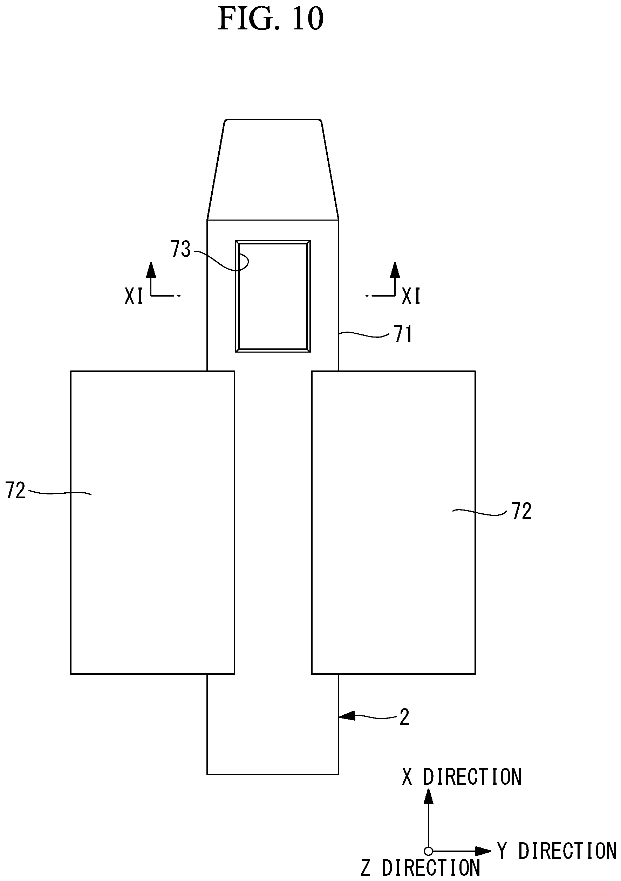

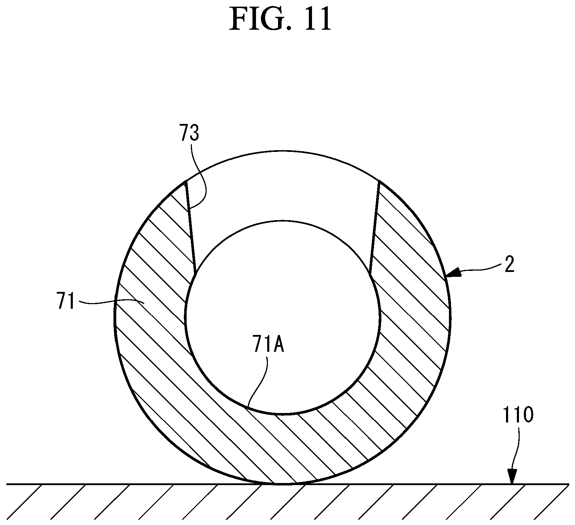

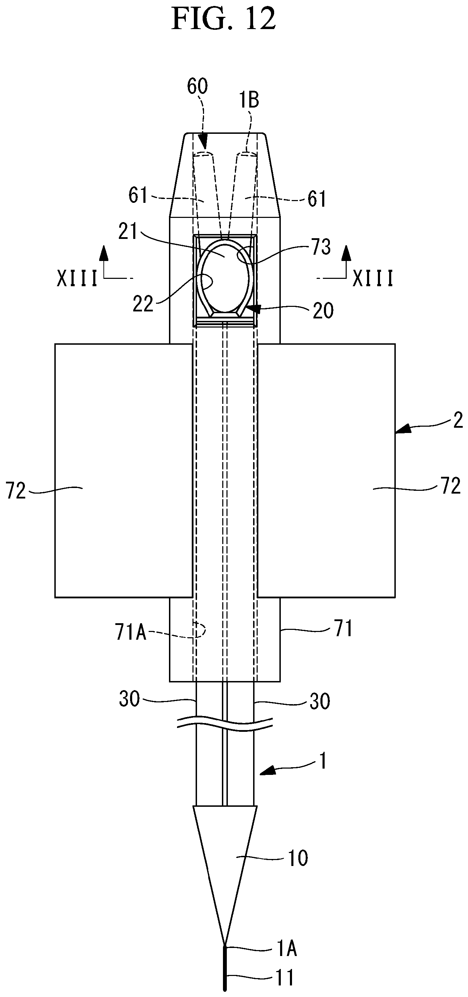

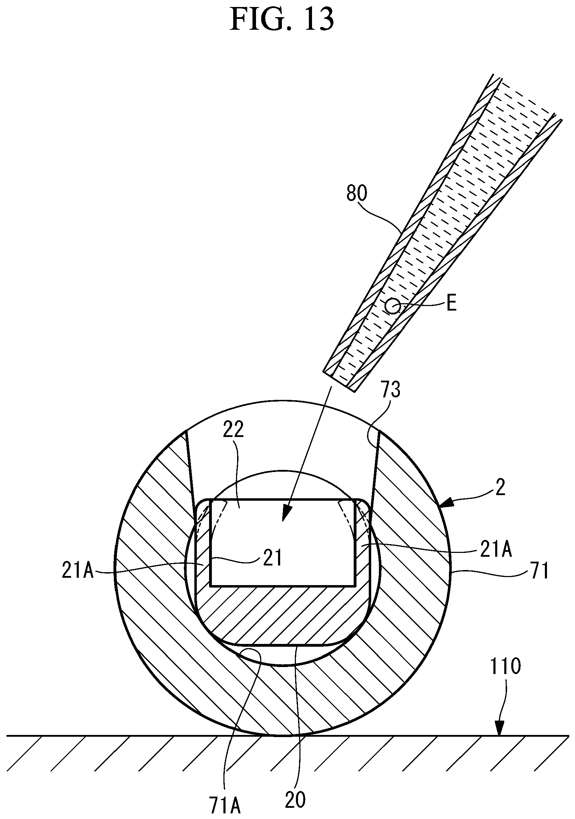

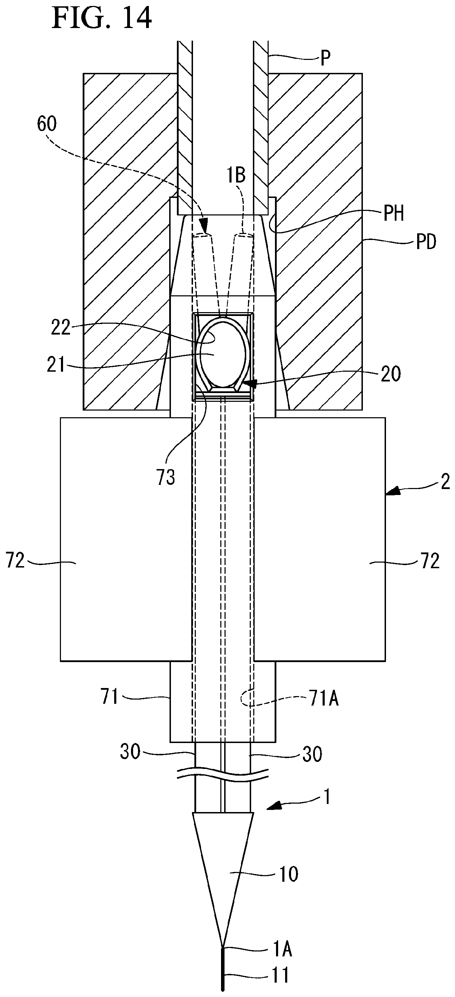

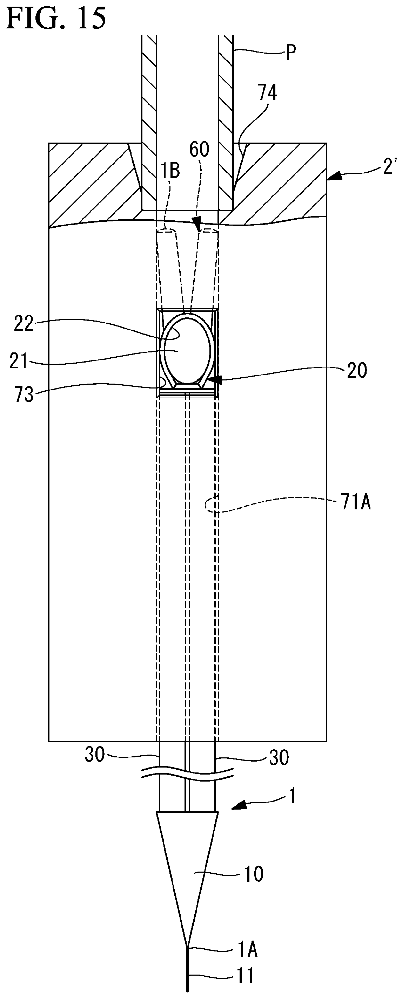

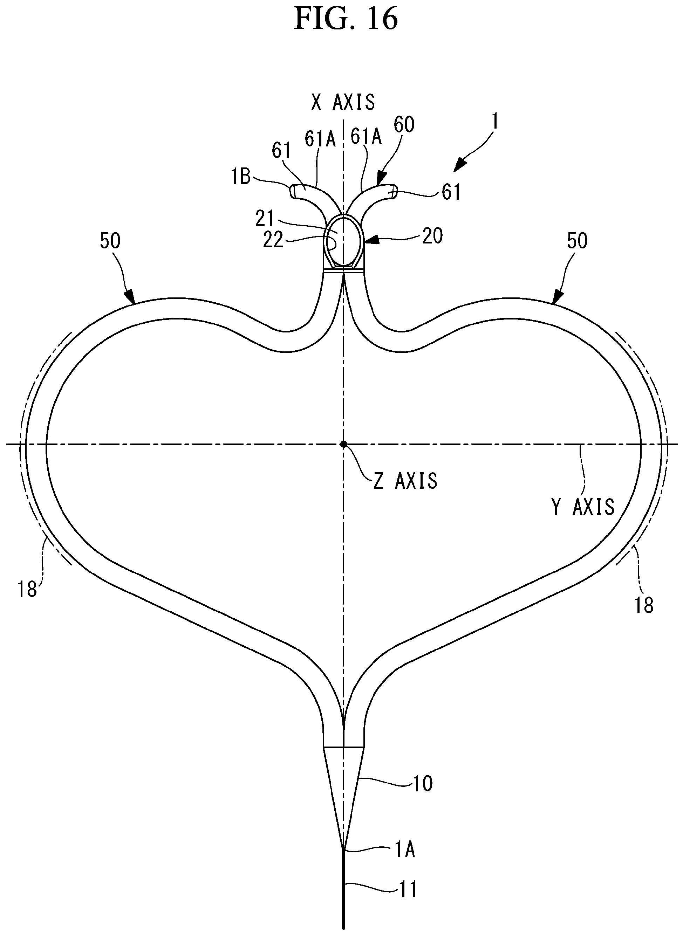

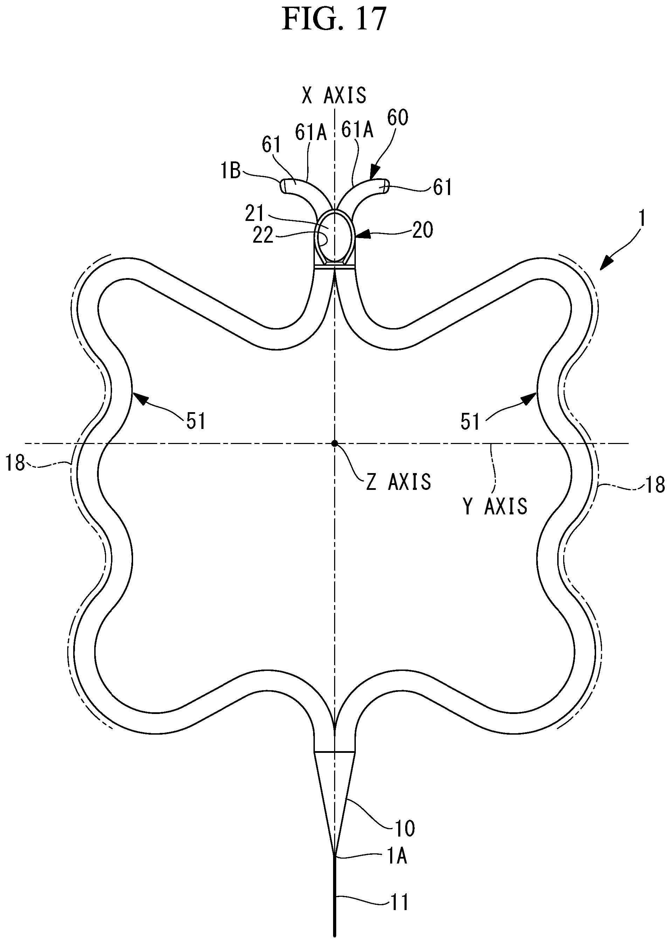

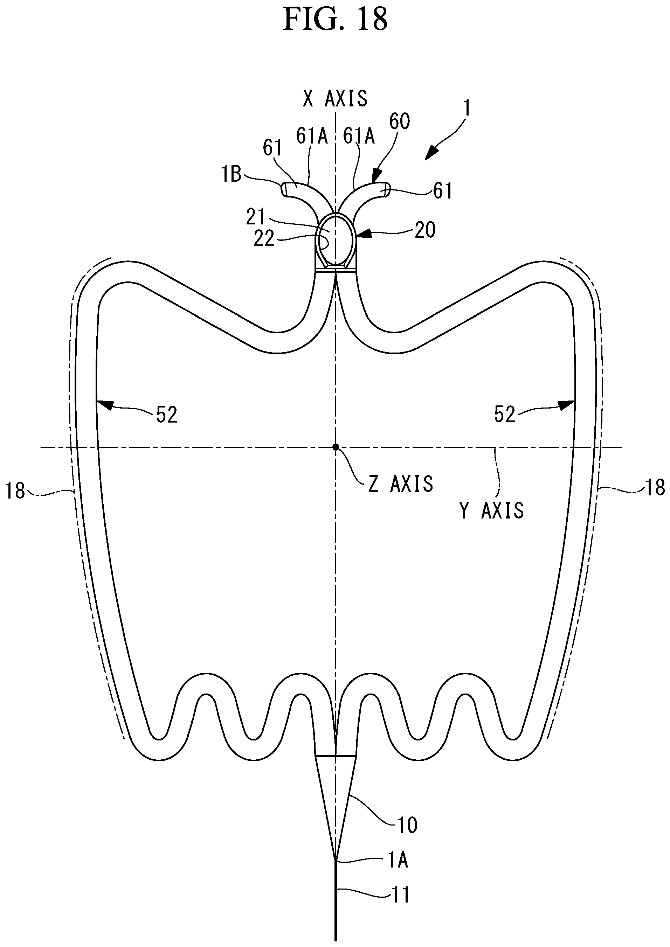

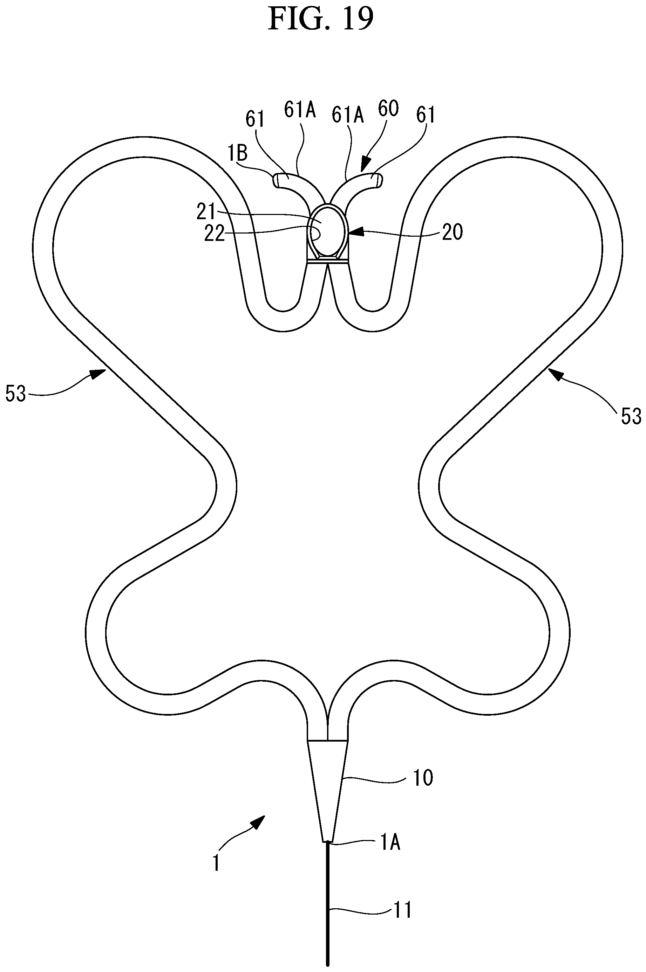

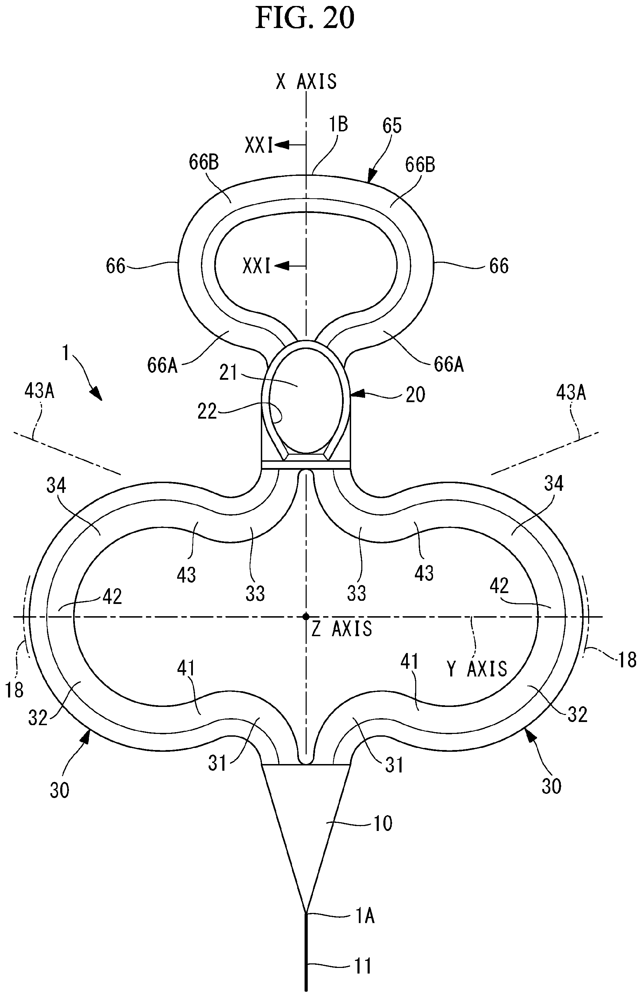

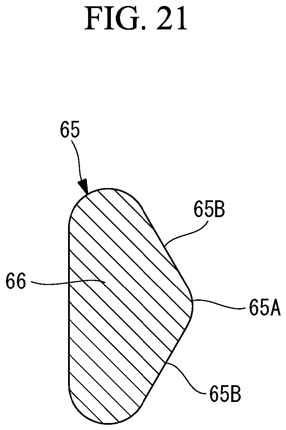

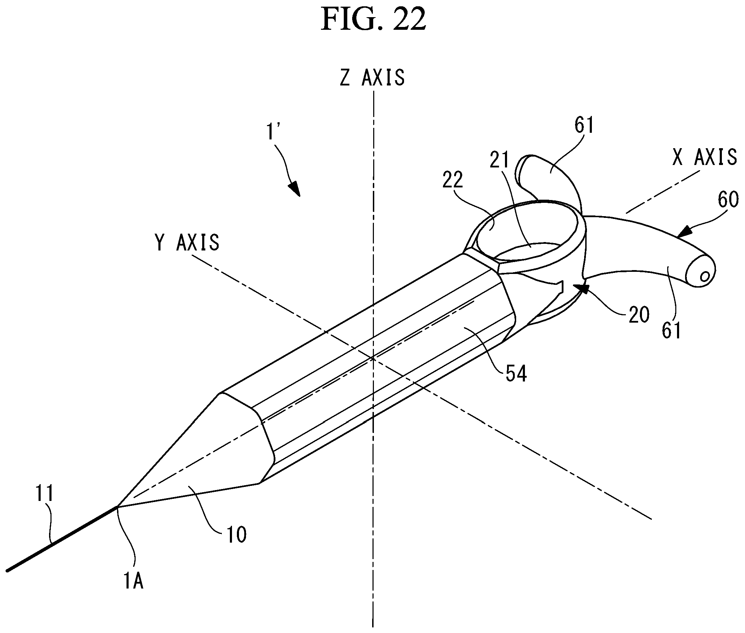

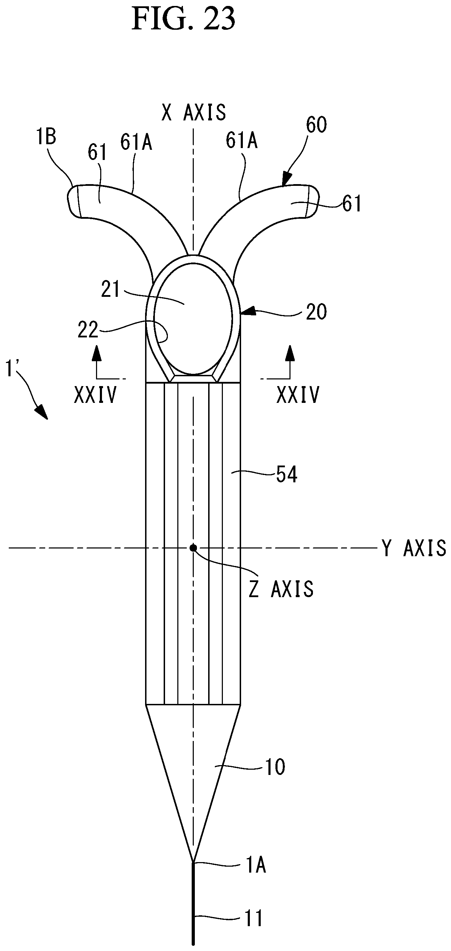

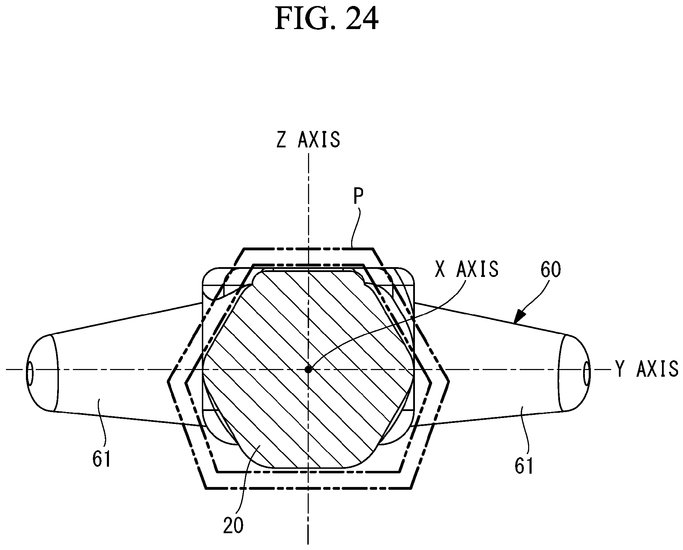

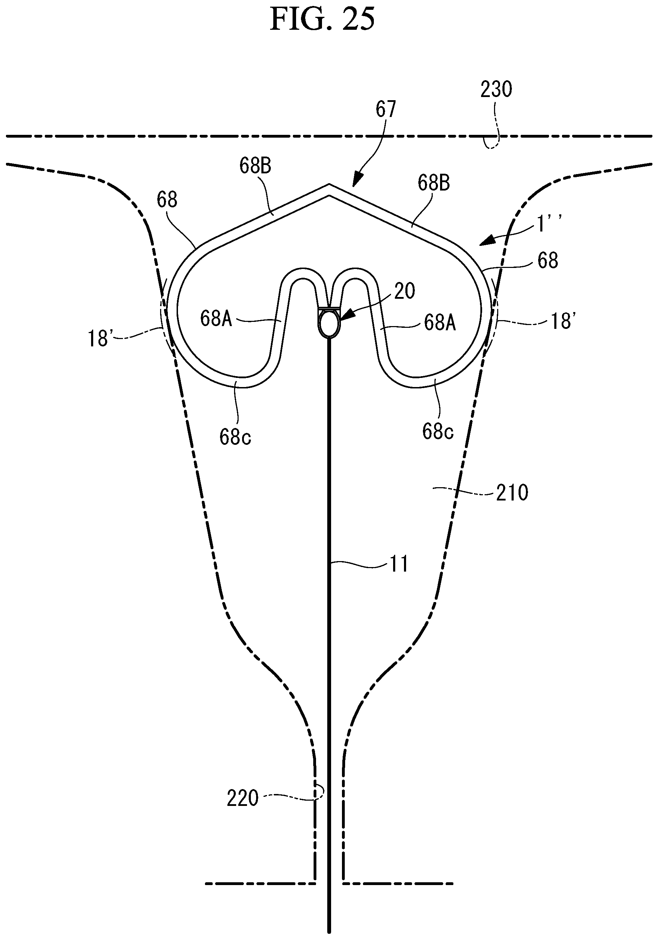

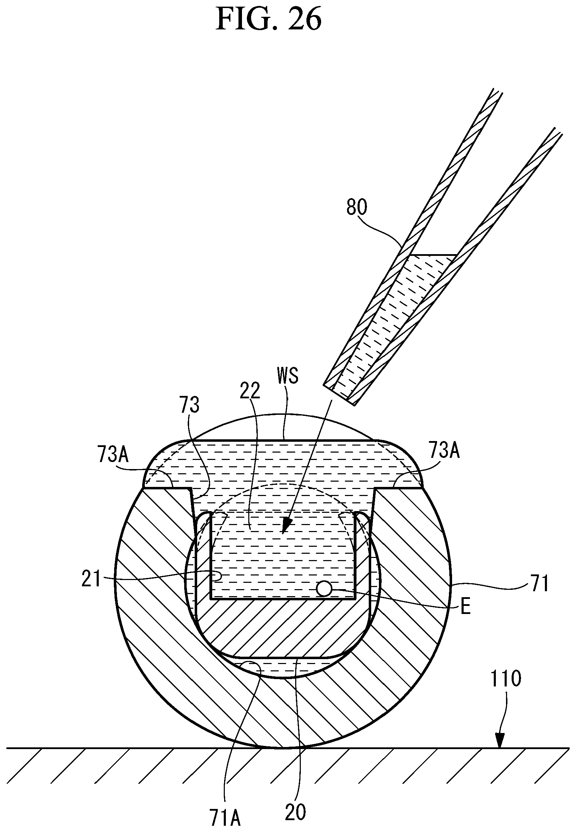

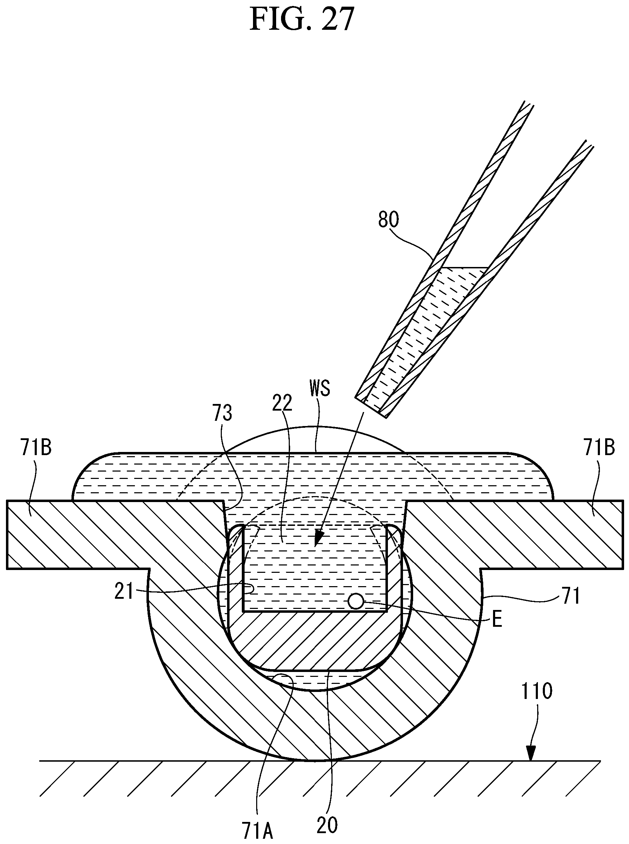

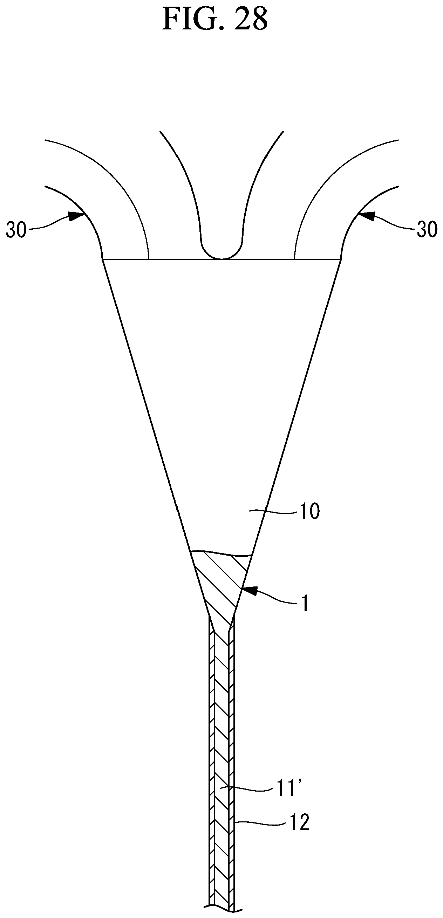

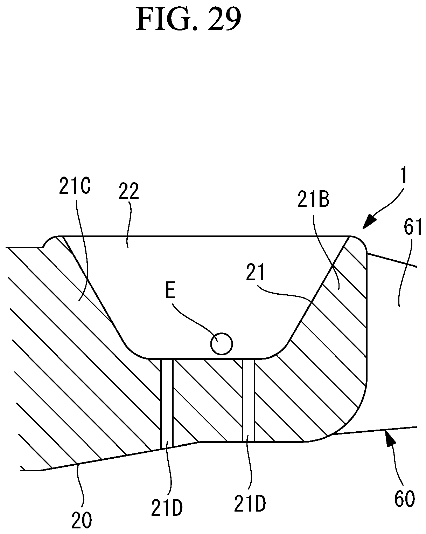

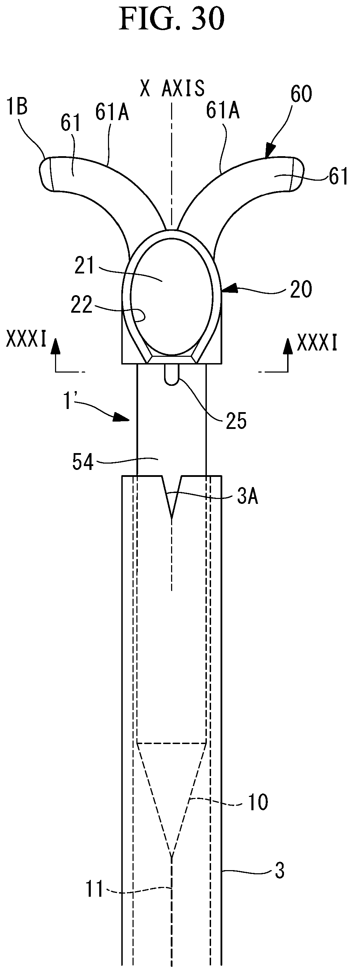

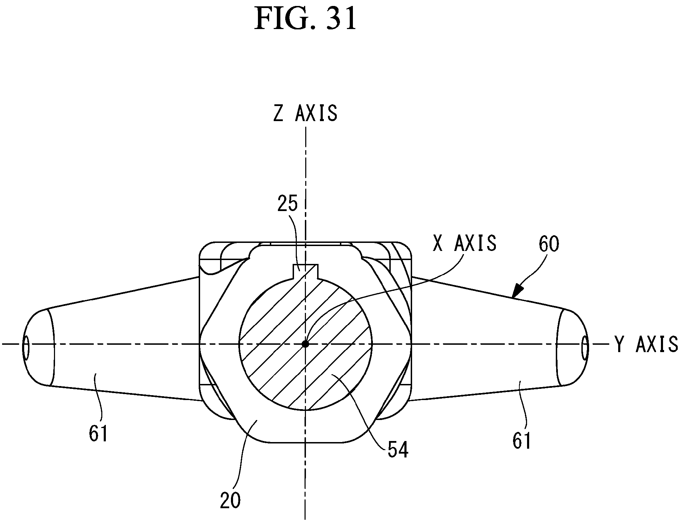

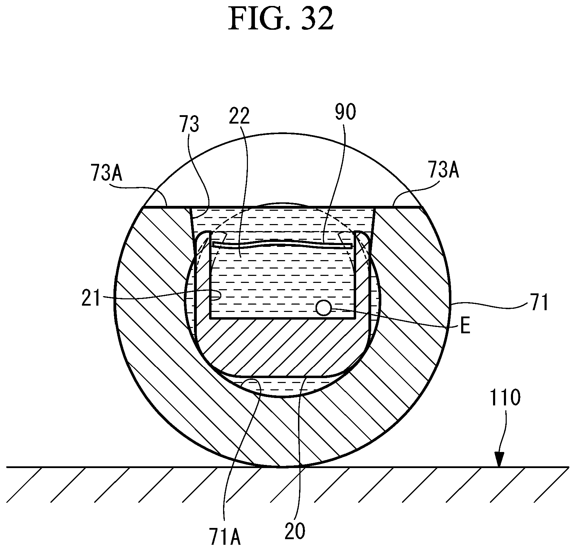

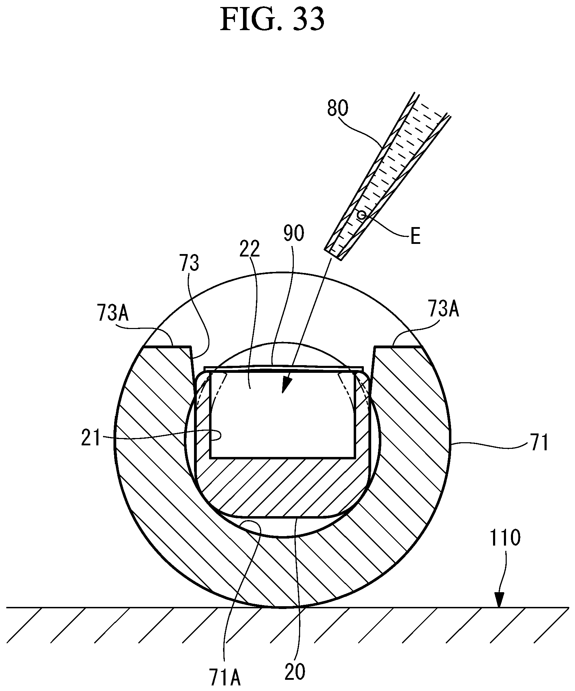

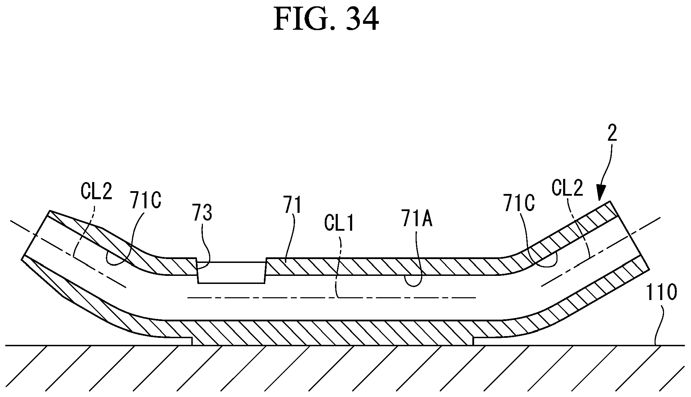

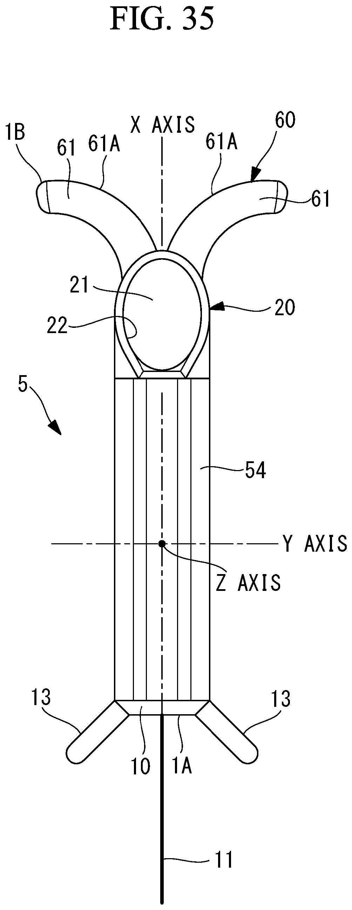

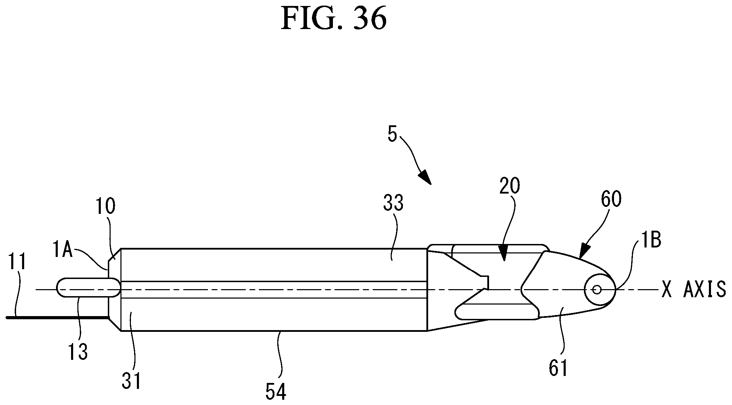

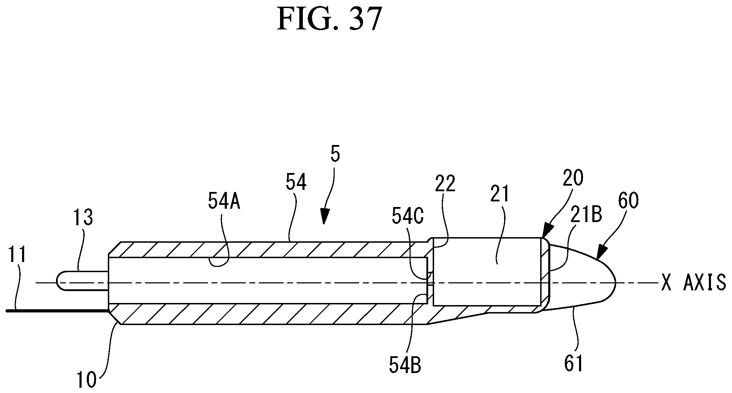

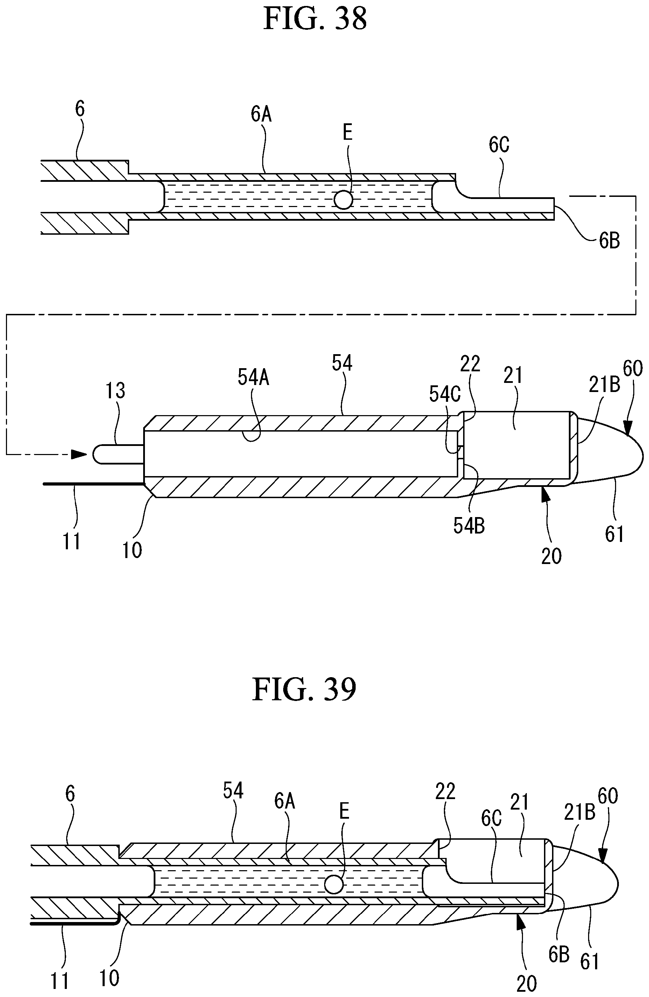

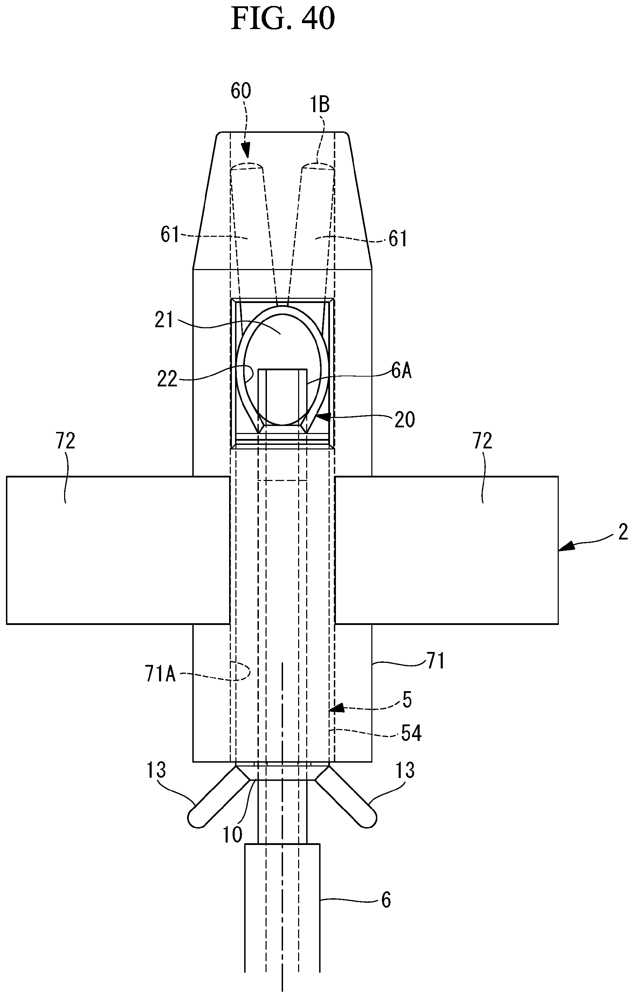

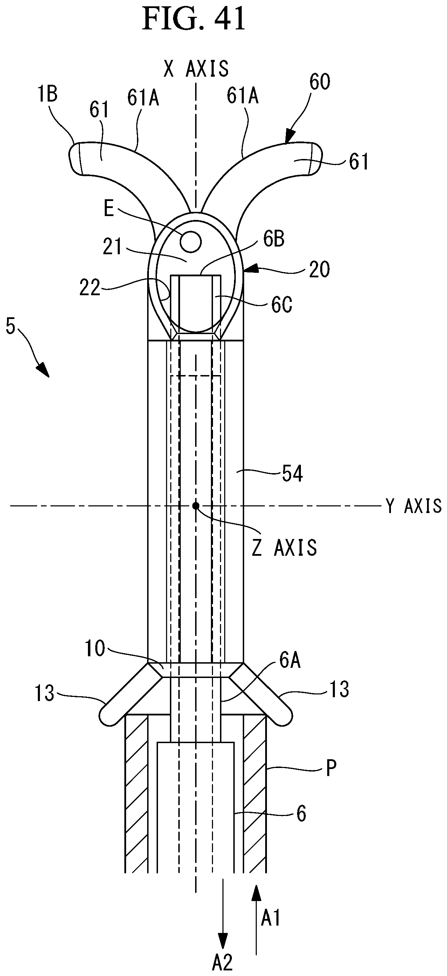

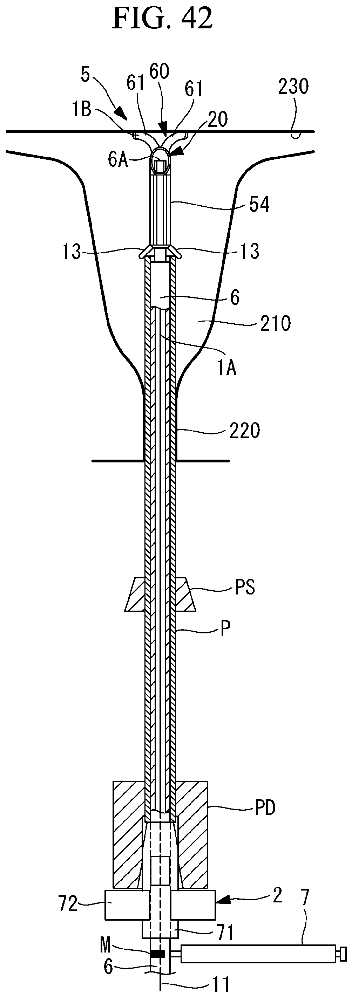

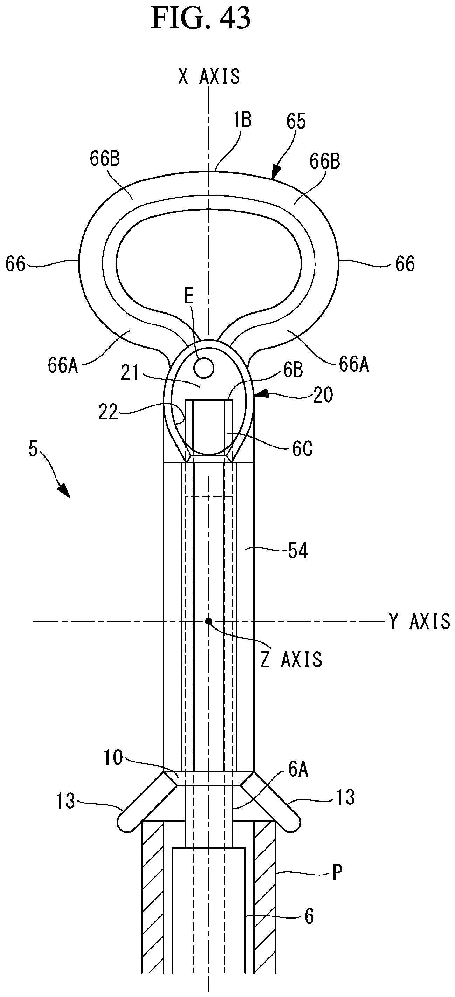

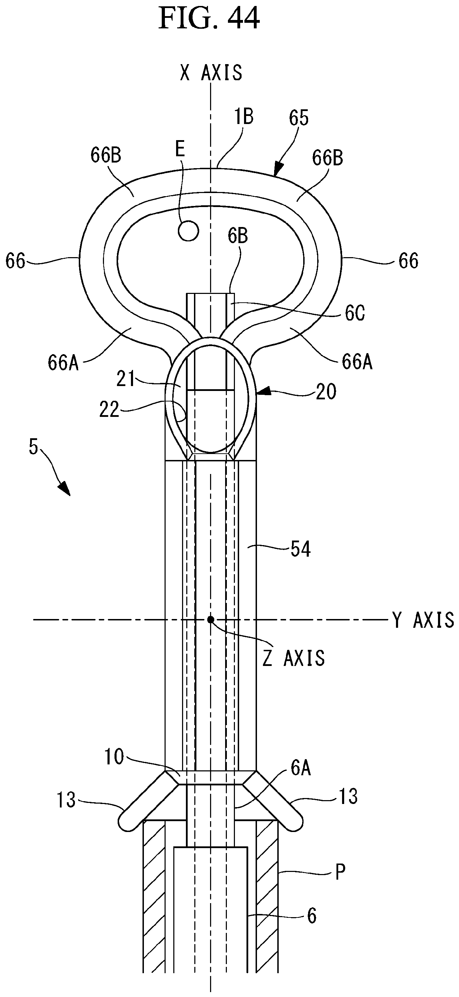

{Fig. 1} Fig. 1 is a perspective view of an embryo transfer device of an embryo transfer device kit of a first embodiment.
{Fig. 2} Fig. 2 is a plan view of the embryo transfer device of the first embodiment.
{Fig. 3} Fig. 3 is a side view of the embryo transfer device of the first embodiment.
{Fig. 4} Fig. 4 is a sectional view of the embryo transfer device in the tube of the first embodiment.
{Fig. 5} Fig. 5 is a partial sectional view of the tube and a stylet of the embryo transfer device kit in the first embodiment.
{Fig. 6} Fig. 6 is a partial sectional view of the embryo transfer device kit of the first embodiment.
{Fig. 7} Fig. 7 is a partial sectional view of the embryo transfer device kit of the first embodiment.
{Fig. 8} Fig. 8 is a plan view of the embryo transfer device of the first embodiment.
{Fig. 9} Fig. 9 is a perspective view of a connection component of the embryo transfer device kit of the first embodiment.
{Fig. 10} Fig. 10 is a plan view of the connection component of the first embodiment.
{Fig. 11} Fig. 11 is a sectional view taken along line XI-XI in Fig. 10.
{Fig. 12} Fig. 12 is a plan view of the connection component accommodating the embryo transfer device in the first embodiment.
{Fig. 13} Fig. 13 is a sectional view of the connection component accommodating the embryo transfer device in the first embodiment.
{Fig. 14} Fig. 14 is a partial sectional plan view showing the first embodiment, showing the connection component accommodating the embryo transfer device and a proximal end portion of the tube.
{Fig. 15} Fig. 15 is a partial sectional plan view showing a first modified example of the first embodiment, showing the connection component accommodating the embryo transfer device and the proximal end portion of the tube.
{Fig. 16} Fig. 16 is a plan view of an embryo transfer device in a second modified example of the first embodiment.
{Fig. 17} Fig. 17 is a plan view of an embryo transfer device in a third modified example of the first embodiment.
{Fig. 18} Fig. 18 is a plan view of an embryo transfer device in a fourth modified example of the first embodiment.
{Fig. 19} Fig. 19 is a plan view of an embryo transfer device in a fifth modified example of the first embodiment.
{Fig. 20} Fig. 20 is a plan view of an embryo transfer device in a sixth modified example of the first embodiment.
{Fig. 21} Fig. 21 is a cross section of a guide part of the sixth modified example of the first embodiment.
{Fig. 22} Fig. 22 is a perspective view of an embryo transfer device of an embryo transfer device kit of a second embodiment.
{Fig. 23} Fig. 23 is a plan view of the embryo transfer device of the second embodiment.
{Fig. 24} Fig. 24 is a sectional view taken along line XXIV-XXIV in Fig. 23.
{Fig. 25} Fig. 25 is a plan view of an embryo transfer device of an embryo transfer device kit of a third embodiment.
{Fig. 26} Fig. 26 is a sectional view of a connection component accommodating an embryo transfer device in a seventh modified example of the first embodiment.
{Fig. 27} Fig. 27 is a sectional view of a connection component accommodating an embryo transfer device in an eighth modified example of the first embodiment.
{Fig. 28} Fig. 28 is a partial sectional plan view of an embryo transfer device in a ninth modified example of the first embodiment.
{Fig. 29} Fig. 29 is an X-direction sectional view of an embryo accommodation portion of an embryo transfer device in a tenth modified example of the first embodiment.
{Fig. 30} Fig. 30 is a plan view of an embryo transfer device and a pusher in a modified example of the second embodiment.
{Fig. 31} Fig. 31 is a sectional view taken along line XXXI-XXXI in Fig. 30.
{Fig. 32} Fig. 32 is a sectional view of a connection component accommodating an embryo transfer device in an eleventh modified example of the first embodiment.
{Fig. 33} Fig. 33 is a sectional view of a connection component accommodating an embryo transfer device in a twelfth modified example of the first embodiment.
{Fig. 34} Fig. 34 is an X-axis sectional view of a connection component in a thirteenth modified example of the first embodiment.
{Fig. 35} Fig. 35 is a plan view of an embryo transfer device of an embryo transfer device kit of a fourth embodiment.
{Fig. 36} Fig. 36 is a side view of the embryo transfer device of the fourth embodiment.
{Fig. 37} Fig. 37 is a sectional view of the embryo transfer device of the fourth embodiment.
{Fig. 38} Fig. 38 is a sectional view of the embryo transfer device and a pusher of the embryo transfer device kit of the fourth embodiment.
{Fig. 39} Fig. 39 is a sectional view of the embryo transfer device and the pusher of the embryo transfer device kit of the fourth embodiment.
{Fig. 40} Fig. 40 is a plan view of the embryo transfer device, a connection component, and the pusher of the embryo transfer device kit of the fourth embodiment.
{Fig. 41} Fig. 41 is a partial sectional plan view of the embryo transfer device, the pusher, and a tube of the embryo transfer device kit of the fourth embodiment.
{Fig. 42} Fig. 42 is a partial sectional plan view of the embryo transfer device, the connection component, the pusher, and the tube of the embryo transfer device kit of the fourth embodiment.
{Fig. 43} Fig. 43 is a partial sectional plan view of an embryo transfer device, a pusher, and a tube of a modified example of the embryo transfer device kit of the fourth embodiment.
{Fig. 44} Fig. 44 is a partial sectional plan view of an embryo transfer device, a pusher, and a tube of another modified example of the embryo transfer device kit of the fourth embodiment.

### {Description of Embodiments}

Hereinafter, an embryo transfer device kit according to a first embodiment will be described with reference to the drawings. As shown in Figs. 6 and 7, the embryo transfer device kit of the first embodiment includes an embryo transfer device 1, a connection component 2, a tube P, a pusher 3 such as a push rod, and a stylet 4.

First, an example of the embryo transfer device 1 will be described with reference to the drawings. The embryo transfer device 1 is inserted into a uterine cavity 210 using the tube P inserted into a uterine cervix 220 (Fig. 4, Fig. 7, etc.). In the present embodiment, the uterine cervix 220 mainly intends a cervical canal between an internal os and an external cervical os. In the present embodiment, the embryo transfer device 1 is indwelled in the uterine cavity 210 for 48 hours or more. The indwelling may be performed for other durations such as several hours or more, 12 hours or more, 24 hours or more, 72 hours or more, etc.

The embryo transfer device 1 is used to implant an embryo E by indwelling the embryo E (Fig. 13) in the uterine cavity 210. What the embryo E of the present embodiment means shall also include a fertilized egg, a fertilized oocyte, and a blastocyst. The embryo transfer device 1 of the present embodiment is for maintaining a state in which the embryo E can contact or is in contact with an endometrium of the uterine cavity 210 and the like in a uterus, and increasing an implantation probability of the embryo E. For implantation, a state in which the embryo E is attached to the endometrium, a state in which the embryo E dives into the endometrium, etc. occur. In one example, the embryo transfer device 1 assists the embryo E to be in a state of being attached to the endometrium. For this reason, in the present embodiment, the embryo transfer device 1 retains the embryo E in a space surrounded by a recess 21 of an embryo accommodation portion 20 described later and the endometrium.

The embryo transfer device 1 is preferably configured so as not to apply unnecessary force or influence to the uterus as much as possible. For example, reduction of force applied to the endometrium from the embryo transfer device 1 disposed in the uterine cavity 210 is expected to contribute to success of implantation. Naturally, it is also important that damage to the uterine cervix 220, the uterine cavity 210, a patient's body, a patient's mind, etc. at the time of insertion/withdrawal of the embryo transfer device 1 is small. On the other hand, during movements of internal organs such as the uterus, movements of the body equipped with the uterus, etc., the embryo transfer device 1 must be reliably indwelled in the uterine cavity 210, and the state in which the embryo E can contact or is in contact with the endometrium for implantation must be maintained. As for these conditions, when one condition is improved, other conditions tend to be lowered. For example, a contraceptive device is reliably indwelled in the uterine cavity 210, but adoption of its configuration has a high possibility of lowering the success rate of implantation because relatively large force is applied to various positions in the uterine cavity 210.

A configuration of the embryo transfer device 1 of the present embodiment shown in Figs. 1 to 5 will be described below. The embryo transfer device 1 has a proximal end 1A and a distal end 1B, and is inserted into the uterine cavity 210 from the distal end 1B using the tube P inserted through a uterine os into the uterine cervix 220 and the uterine cavity 210 as shown in Fig. 5, etc. In the present embodiment, the proximal end 1A and the distal end 1B are a proximal end and a distal end of the embryo transfer device 1 when disposed in the tube P as shown in Fig. 6, etc. The embryo transfer device 1 has a proximal end portion 10 forming at least a portion of the proximal end 1A, and the embryo accommodation portion 20 provided on the side of the distal end 1B and accommodating the embryo E. In description of the present embodiment, as shown in Figs. 1 to 3, an X axis passes through the proximal end portion 10 and the embryo accommodation portion 20, a Y axis orthogonal to the X axis extends in a direction in which expandable frame parts 30 described later expand, and a Z axis is orthogonal to the X axis and the Y axis. In the present embodiment, it can be said that the Y axis extends in a direction orthogonal to an opening direction of an opening 22 of the recess 21 and a direction in which the X axis extends. Note that in the present embodiment, the distal end 1B is an end of an embryo accommodation portion guide 60 extending from the embryo accommodation portion 20, but there may be a case were the distal end 1B is the embryo accommodation portion 20.

The embryo transfer device 1 of the present embodiment has two expandable frame parts 30 connecting the proximal end portion 10 and the embryo accommodation portion 20. One end of each expandable frame part 30 is connected to the proximal end portion 10, and the other end is connected to the embryo accommodation portion 20. Each expandable frame part 30 is formed of a material having rubber-like elasticity. Examples of the material having rubber-like elasticity are rubber materials such as silicone rubber, other elastic materials, etc.

Other types of rubber materials can also be used as the rubber material. Rubber hardness of the rubber material of the present embodiment is in a range of 90 points or less when measured with a type A durometer in a state of 23±2° C., but is not limited thereto. For example, the rubber hardness of the rubber material of the present embodiment is in a range of 10 to 90 points when measured with an Asker rubber durometer type A manufactured by Kobunshi Keiki Co., Ltd. in a state of 23±2° C. This value of 10 to 90 points means durometer hardness of JIS K 6253. Since each expandable frame part 30 etc. of the present embodiment is thin, an approximate value of the type A durometer (Asker rubber durometer type A) may be measured with a durometer sold under a product name GS-680sel manufactured by Teclock Co., Ltd., and the rubber hardness of each expandable frame part 30 may be determined using the approximate value. A micro rubber durometer manufactured by Kobunshi Keiki Co., Ltd. of a product name MD-1capa may be used instead of GS-680sel.

In the present embodiment, assuming that Young's modulus at the time of contraction of a myometrium under the endometrium is around 10 MPa, the rubber hardness is set as described above so that Young's modulus of a material of each expandable frame part 30 becomes equal to or less than that. The setting may exert an effect that each expandable frame part 30 is not recognized as a foreign object in the uterine cavity 210. Note that Young's modulus of a blood vessel under the endometrium may be lower than the above Young's modulus. Considering Young's modulus of the blood vessel etc., it may be better that Young's modulus of the material of each expandable frame part 30 is lower. In this case, the rubber hardness of the material of each expandable frame part 30 is set to 70 points or less, 65 points or less, 60 points or less, 55 points or less, or 50 points or less.

Note that when it is difficult to measure the rubber hardness of each expandable frame part 30 etc. with the Asker rubber durometer type A, a sheet of the same material having a thickness of 6 mm to 10 mm can be molded using a mold, hardness of the sheet can be measured with the Asker rubber durometer type A in a state of 23±2° C., and the measurement result can be taken as the rubber hardness of each expandable frame part 30.

Preferably, the proximal end portion 10 and the embryo accommodation portion 20 are also formed of a material having Young's modulus (rubber hardness) in the same range. In the present embodiment, the proximal end portion 10, the embryo accommodation portion 20, and the two expandable frame parts 30 are integrally formed using a mold, and the proximal end portion 10, the embryo accommodation portion 20, and the two expandable frame parts 30 consist of the same material. The structure of the embryo transfer device 1 is not limited to this example.

The two expandable frame parts 30 are elastically deformed into a state in which they are close to each other when accommodated in the tube P for insertion into the uterine cavity 210 (Fig. 6). At this time, in the present embodiment, parts of the two expandable frame parts 30 come into contact with each other. When taken out from the tube P, the two expandable frame parts 30 expand in a direction along the Y axis by restoring force of the elastic deformation (Figs. 7 and 8). In the present embodiment and the following embodiments, the "direction along the X axis", the "direction along the Y axis", and the "direction along the Z axis" may be referred to as an "X direction", a "Y direction", and a "Z direction", respectively.

In the present embodiment, when taken out from the tube P, the two expandable frame parts 30 move so that at least intermediate portions in the length direction separate from each other and separate from the X axis by the restoring force of the elastic deformation, and expand by the movement. Since the X axis is along a central axis on the distal end side of the tube P in the tube P, it can be said that the intermediate portions move so as to separate from a central axis CL (Fig. 6). Also, in the present embodiment, it can be said that the embryo accommodation portion 20 opens in a direction along an axis (Z axis) orthogonal to the central axis CL when taken out from the tube P. Note that in the present embodiment, when the embryo transfer device 1 is disposed in the tube P as shown in Fig. 6, the direction in which the X axis passing through the proximal end portion 10 and the embryo accommodation portion 20 of the embryo transfer device 1 extends coincides or substantially coincides with the central axis CL, but it is not limited thereto. In the present embodiment, the substantial coincidence means a case where an angle formed by the direction in which the X axis extends and the central axis CL is 10° or less, and preferably the angle is 5° or less. Note that even if the angle is larger than the above angle, the same or a similar effect can be achieved. Note that it is allowed that the two expandable frame parts 30 also move in a direction along the Z axis etc. at the time of the expansion. That is, the movement of the two expandable frame parts 30 at least in the Y direction as described above is expansion in the Y direction. Note that it is preferable that movement of the two expandable frame parts 30 in the Y direction at the time of expansion is larger than movements in the X direction and the Z direction, and in this case, it can be said that the two expandable frame parts 30 mainly move in the Y direction at the time of expansion.

Here, a typical example of the tube P is a plastic tube. The outer diameter of the tube P is preferably 2.5 mm or less, more preferably 2.0 mm or less, and further preferably 1.5 mm or less, in order to reduce the aforementioned damage to the uterus at the time of insertion/withdrawal. For this reason, an inner diameter of the tube P is 2.3 mm or less, 1.8 mm or less, 1.3 mm or less, etc. The tube P may be referred to as a guide, a sheath, etc., and may be referred to as a catheter, but tubes of other names can also be used.

In order to dispose the embryo transfer device 1 in a hollow section of such a thin tube P, a cross-sectional area of each expandable frame part 30 becomes small. For example, when the inner diameter (diameter of the hollow section) of the tube P is 1.8 mm, each of the two expandable frame parts 30 is disposed in a narrow area of 1/2 of the cross-sectional area of the hollow section of the tube P. For example, as shown in Fig. 4, as a cross-sectional shape of each expandable frame part 30, an isosceles triangle, a shape close to an isosceles triangle, etc. can be adopted. For this reason, the cross section of each expandable frame part 30 has two sides 30B of substantially straight lines respectively extending obliquely from one side and the other side in the Z direction toward a top portion 30A in the Y direction as shown in Fig. 4. The cross-sectional shape of each expandable frame part 30 can be a semicircular shape, and can be a shape between them or a shape similar to them. The configuration can make the cross-sectional area of each expandable frame part 30 as large as possible in the narrow area. This contributes to disposing the embryo accommodation portion 20 at a desired place as described later.

The proximal end portion 10 only needs to have a cross-sectional area equal to or less than that of the hollow section of the tube P. In the present embodiment, the proximal end portion 10 has a cylindrical shape or a conical shape with a maximum diameter smaller than the inner diameter of the tube P by about 0.1 mm. The proximal end portion 10 may have other shapes. In the present embodiment, an end of a string 11 for withdrawal is fixed to the proximal end portion 10. In the present embodiment, it can be said that the proximal end portion 10 is a portion pushed by the pusher 3 described later. In another embodiment, the proximal end portion 10 is a portion where the string 11 comes out from the embryo transfer device 1. In still another embodiment, the string 11 may not come out from the proximal end portion 10, and the proximal end portion 10 may not be pushed by the pusher 3.

The embryo accommodation portion 20 opens in the direction along the Z axis. In the present embodiment, as shown in Fig. 1, Fig. 13, etc., the embryo accommodation portion 20 has the recess 21 recessed in the direction along the Z axis, and the opening direction of the opening 22 of the recess 21 is the direction along the Z axis. The embryo E is accommodated in the recess 21. In the present embodiment, the opening direction of the opening 22 is a direction completely along the Z axis, but if an angle formed by the opening direction and the Z axis is 60° or less, preferably 45° or less, and more preferably 30° or less, it leads to achievement of effects described later, and it can be said that the opening 22 opens in the direction along the Z axis. Also, there are individual differences in the shape of the uterine cavity 210, and the embryo transfer device 1 is formed of a material having rubber-like elasticity. For this reason, the opening direction of the embryo accommodation portion 20 in the uterine cavity 210 is adjusted in accordance with the shape of the uterine cavity 210. The adjustment of the opening direction is useful for maintaining a state in which the endometrium exists in the opening direction. In the present embodiment, the opening direction is a direction orthogonal to a virtual plane along an edge of the opening 22. Even if the angle formed by the opening direction and the Z axis exceeds 60°, the effects described later may be expected depending on the shape etc. of the uterine cavity 210. Thus, since the embryo accommodation portion 20 opens in the direction along the Z axis in a no-load state, when the embryo transfer device 1 is disposed in the uterine cavity 210, the opening 22 contacts or comes close to the endometrium, and the state in which the embryo E in the embryo accommodation portion 20 can contact or is in contact with the endometrium is maintained.

The shape of each expandable frame part 30 will be described below in the no-load state where no load is applied to each expandable frame part 30. Note that the no-load state is, for example, a state in which the embryo transfer device 1 is placed on a horizontal surface 100 as shown in Fig. 3, and only gravity is applied to the embryo transfer device 1. In Fig. 3, the one end side, the intermediate portion, etc. of each expandable frame part 30 are in contact with the horizontal surface 100.

In the no-load state, each expandable frame part 30 has a first bent portion 31 curving in a direction away from the X axis from the one end toward the other end, and a second bent portion 32 disposed closer to the other end than the first bent portion 31 and bent in a direction approaching the X axis from the one end toward the other end.

Also, in the no-load state, each expandable frame part 30 has a third bent portion 33 disposed closer to the other end than the second bent portion 32 and bent in a direction away from the X axis from the other end toward the one end. Also, in the no-load state, each expandable frame part 30 has a fourth bent portion 34 disposed closer to the other end than the second bent portion 32 and disposed closer to the one end than the third bent portion 33, and bent in a direction approaching the X axis from the one end toward the other end. The second bent portion 32 and the fourth bent portion 34 may be a continuous bent portion.

In each embodiment, a portion between the first bent portion 31 and the second bent portion 32 is referred to as a first connection portion 41, a portion between the second bent portion 32 and the fourth bent portion 34 is referred to as a second connection portion 42, and a portion between the fourth bent portion 34 and the third bent portion 33 is referred to as a third connection portion 43.

As shown in Fig. 6, when the embryo transfer device 1 is disposed inside the tube P, the curvature (degree of bending) of each of the bent portions 31, 32, 33, and 34 becomes gentle, and in some cases, the bent portions may become substantially straight. When the embryo transfer device 1 is disposed in the tube P, the first bent portion 31, the second bent portion 32, the fourth bent portion 34, the third bent portion 33, the embryo accommodation portion 20, and the embryo accommodation portion guide 60 are arranged in this order from the proximal end 1A side toward the side of the distal end 1B. Note that the entire embryo transfer device 1 may be completely stored in the tube P, and a part of the embryo transfer device 1, for example, a part of the proximal end portion 10 or a part of the embryo accommodation portion guide 60 may be exposed from the tube P.

For example, when a user disposes the embryo transfer device 1 in the uterine cavity 210, first, the user inserts the distal end side of the tube P from the external cervical os into the uterine cervix 220, and disposes the distal end side of the tube P in the uterine cavity 210 (Fig. 5). At this time, in the present embodiment, the stylet 4 is disposed in the tube P so as to be inserted through the tube P as shown in Fig. 5. Note that the user at this time is, for example, a practitioner. Also, if the tube P is provided with a stopper PS clinging to an outer peripheral surface thereof, an insertion amount of the tube P inserted into the uterine cervix 220 by the user is limited. In one example, the stopper PS contacts the external cervical os, whereby the insertion amount of the tube P is limited. The stopper PS consists of a material having rubber-like elasticity such as silicone rubber, plastic, etc., and an attachment position of the stopper PS with respect to the tube P is adjustable in a direction along the central axis of the tube P.

In one example, the user disposes the distal end sides of the tube P and the stylet 4 in the uterine cavity 210 while checking positions of distal ends of the tube P and the stylet 4 by ultrasonic observation. For this purpose, preferably, a visibility improving portion is provided on the distal end side of the tube P and/or the distal end side of the stylet 4, whereby the user can easily recognize the positions of the tube P and/or the stylet 4 in the ultrasonic observation. The visibility improving portion is provided by changing a material on the distal end side of the tube P or the stylet 4, providing a characteristic shape such as minute unevenness or a groove on a surface on the distal end side, embedding bubbles, microspheres, minute particles, etc. inside the distal end side, or by other methods. Note that the visibility improving portion may be provided in the embryo accommodation portion 20, the expandable frame part 30, etc. In this case, the user can dispose the embryo transfer device 1 in the uterine cavity 210 while checking positions of the embryo accommodation portion 20, the expandable frame part 30, etc. by ultrasonic observation. As the microspheres and the minute particles, known small spheres and particles such as hollow beads can be naturally used. A material of the hollow beads is glass, plastic, etc. In order to provide the bubbles, naturally it is also possible to embed a known porous member such as porous silicon in the tube P, the stylet 4, the embryo accommodation portion 20, the expandable frame part 30, etc.

Next, the user pulls out the stylet 4 from the tube P, and body fluid tends to enter the tube P at this time. The body fluid can contribute to moving the embryo transfer device 1 smoothly in the tube P as described later.

Next, as described later, the user applies force toward the distal end of the tube P to the embryo transfer device 1 by the pusher 3, thereby moving the embryo transfer device 1 from the connection component 2 to the tube P. Then, the user applies force toward the distal end of the tube P to the embryo transfer device 1 by the pusher 3, thereby the embryo transfer device 1 within the tube P is moved into the uterine cavity 210. At this time, the user may move the tube P in a direction of pulling it out from the uterine cervix 220. When moving the embryo transfer device 1 in the tube P to the uterine cavity 210, the user holds a proximal end (pusher proximal end) side of the pusher 3. In the present embodiment, the pusher 3 which is a push rod has a thin pipe shape made of plastic, metal, etc., but the pusher 3 may be formed of other materials and may have other shapes. In order to reduce movement resistance of the pusher 3 in the tube P, preferably the pusher 3 consists of fluororesin such as PTFE.

Thereby, as shown in Fig. 7, in the uterine cavity 210, the embryo accommodation portion guide 60 comes out first from the tube P, and thereafter the embryo accommodation portion 20 and the third bent portion 33 come out in order. Subsequently, the fourth bent portion 34, the second bent portion 32, the first bent portion 31, and the proximal end portion 10 come out from the tube P in order, and the embryo transfer device 1 is disposed in the uterine cavity 210 (Fig. 8).

After the other ends of the deployable frame parts 30 emerge from the tube P and before the one ends thereof emerge, the two expandable frame parts 30 expand in the Y direction. When expanded in this way, when viewed from the direction along the Z axis, the third connection portion 43 of each expandable frame part 30 is disposed so as to extend mainly in the Y direction (Fig. 2). In one example, an extension direction 43A (Fig. 2) which is a direction in which the third connection portion 43 extends is a direction in which a straight line connecting both terminals of the third connection portion 43 extends. When an angle formed by the extension direction 43A and the Y axis is less than 45° when viewed from the direction along the Z axis, it can be said that the third connection portion 43 extends mainly in the Y direction.

In Fig. 2, the third connection portion 43 extends substantially linearly when viewed from the direction along the Z axis, but the third connection portion 43 may be curved when viewed from the Z direction, and even in this case, the direction in which the straight line connecting the both terminals extends can be determined. Also, there is a case where the third bent portion 33 and the fourth bent portion 34 are directly connected, and there is a case where the third bent portion 33 and the fourth bent portion 34 are connected via a short third connection portion 43. In these cases, a predetermined range including a connection portion between the third bent portion 33 and the fourth bent portion 34 in each expandable frame part 30 functions as the third connection portion 43. The predetermined range is, for example, a range extending obliquely to a deep side of the uterine cavity 210 from the other end toward the one end of each expandable frame part 30. The deep side is a side of the distal end 1B in the direction along the X axis. The third connection portion 43 of the present embodiment also extends obliquely to the deep side of the uterine cavity 210 from the other end toward the one end of each expandable frame part 30.

Other portions of the embryo transfer device 1 also come into contact with the uterus, but in the present embodiment, width direction outer portions 18 (Fig. 2) of the two expandable frame parts 30 constituting both sides in the Y direction in the embryo transfer device 1 come into contact with side walls of the uterine cavity 210. The side walls are typically both walls in the width direction of the uterine cavity 210 in the body as shown in Fig. 8. In the present embodiment, the width direction may be referred to as a width direction of the uterine cavity 210. The contact becomes movement resistance of the width direction outer portion 18 with respect to the uterus. Note that the one end side of the width direction outer portion 18 in each expandable frame part 30 also becomes movement resistance. Note that in the case of the present embodiment, as shown in Fig. 2, a part of the second bent portion 32, a part of the fourth bent portion 34, and the second connection portion 42 may be the width direction outer portion 18. Alternatively, a part of the fourth bent portion 34 and the second connection portion 42 may be the width direction outer portion 18, a part of the second bent portion 32 and the second connection portion 42 may become the width direction outer portion 18, or only the second connection portion 42 may be the width direction outer portion 18. In any case, in the present embodiment, the width direction outer portions 18 which are intermediate portions of the respective expandable frame parts 30 come into contact with both walls in the width direction of the uterine cavity 210.

Thus, the width direction outer portion 18 is disposed at a position farther from the X axis than the embryo accommodation portion 20. For this reason, an effect is obtained in which the embryo storage portion 20 is less likely to be disposed on the outer side in the width direction of the uterine cavity 210. Also, by the configuration, an effect of prevention or reduction of the embryo E being disposed on a fallopian tube side is obtained.

In each expandable frame part 30, the one end is connected to the proximal end portion 10 and the other end is connected to the embryo accommodation portion 20, and expands in the Y direction. For this reason, although each expandable frame part 30 is formed of the thin material having rubber-like elasticity as described above, compared with a case where the embryo accommodation portion 20 is supported by a thin rubber rod extending along the X axis from the proximal end portion 10 and insertable into the tube P, an effect is obtained in which the position of the embryo accommodation portion 20 in the Y direction is easily stabilized. Also, the embryo accommodation portion 20 is in a state of being supported via the pair of third connection portions 43 with respect to the pair of width direction outer portions 18, and each third connection portion 43 extends mainly in the Y direction. Alternatively, as described above, the predetermined range including the connection portion between the third bent portion 33 and the fourth bent portion 34 in each expandable frame part 30 extends mainly in the Y direction. For this reason, the effect that the position of the embryo accommodation portion 20 in the Y direction is easily stabilized is more reliably achieved.

Note that in the present embodiment, in each expandable frame part 30, the one end is connected to the proximal end portion 10 and the other end is connected to the embryo accommodation portion 20, and each expandable frame part 30 expands in the Y direction. By the configuration, the intermediate portions of the pair of expandable frame parts 30 are disposed on both sides in the width direction of the uterine cavity 210, the other end sides of the pair of expandable frame parts 30 are disposed on the deep side of the uterine cavity 210, and the one end sides of the pair of expandable frame parts 30 are disposed on an entrance (uterine os) side of the uterine cavity 210. In the present embodiment, both sides in the width direction of the uterine cavity 210 may be referred to as both side walls.

Thus, the pair of expandable frame parts 30 are disposed along the both side walls, the deep side, and the entrance side of the uterine cavity 210. There is a case where the pair of expandable frame parts 30 are disposed in the uterine cavity 210 so as not to contact the both side walls. In one example, the intermediate portions (width direction outer portions 18) of the pair of expandable frame parts 30 are disposed along the both side walls in the uterine cavity 210, and the one end sides of the pair of expandable frame parts 30 are disposed between the intermediate portions in the width direction of the uterine cavity 210. Also, the other end sides of the pair of expandable frame parts 30 are disposed closer to the deep side of the uterine cavity 210 than the one end sides, and are disposed between the intermediate portions in the width direction of the uterine cavity 210. In the present embodiment, such arrangement of the pair of expandable frame parts 30 may be referred to as a ring arrangement.

In the case of the present embodiment, to describe more accurately, the pair of expandable frame parts 30, the embryo accommodation portion 20, and the proximal end portion 10 are disposed along sides of the both side walls, the deep side, and the entrance sideof the uterine cavity 210, but the range of the pair of expandable frame parts 30 alone can be said to be the above ring arrangement. It is preferable that the pair of expandable frame parts 30 occupy a length of 70% or more, more preferably 80% or more, and further preferably 90% or more in a circumferential direction of the ring, in order to realize stabilization of the position of the embryo accommodation portion 20 in the uterine cavity 210 and reduction of force applied to the endometrium from the embryo transfer device 1.

Each expandable frame part 30 is formed of the thin material having rubber-like elasticity as described above, but the effect that the position of the embryo accommodation portion 20 is likely to be stabilized is obtained by the ring arrangement. That is, when the embryo accommodation portion 20 moves in the Y direction, in one example, force around the center of the ring, for example, is applied to the entirety of each expandable frame part 30. Movement resistance occurs in each expandable frame part 30 due to contact with the uterine cavity 210 etc. For this reason, there is an effect that each expandable frame part 30 which is thin and has rubber-like elasticity can stabilize the position of the embryo accommodation portion 20 by the ring arrangement. Such an effect of the ring arrangement can be obtained even if the angle formed by the extension direction 43A and the Y axis is 45° or more when viewed from the direction along the Z axis.

Also, in the present embodiment, the embryo accommodation portion guide 60 extends from the embryo accommodation portion 20, and the embryo accommodation portion guide 60 becomes resistance when the embryo accommodation portion 20 moves in the Y direction. As one of its effects, it is possible to stabilize the position of the embryo accommodation portion 20.

Also, in the present embodiment, the extension direction 43A is inclined to the deep side of the uterine cavity 210 from the other end toward the one end of each expandable frame part 30, and the distal end 1B side of the pair of expandable frame parts 30 has a square shoulder shape. On the other hand, the extension direction 43A may be inclined to the entrance side of the uterine cavity 210 (the side of the proximal end 1A in the direction along the X axis) from the other end toward the one end of each expandable frame part 30, and the distal end 1B side of the pair of expandable frame parts 30 may have a sloping shoulder shape. Even in the case of having the sloping shoulder shape, the effect of the ring arrangement is obtained.

Also, the effect of stabilization of the position of the embryo accommodation portion 20 mainly in the X direction can also be expected by the ring arrangement. In order to stabilize the position of the embryo accommodation portion 20 mainly in the X direction, it is also conceivable to set the angle formed by the extension direction 43A and the Y axis when viewed from the direction along the Z axis to 45° or more.

Considering individual differences in the shape, size, state, etc. of the uterine cavity 210, multiple types of embryo transfer devices 1 having different lengths, shapes, etc. of the expandable frame part 30 may be prepared. For example, expandable frame parts 50, 51, 52, 53 in Figs. 16 to 19 may be adopted instead of the expandable frame part 30. The expandable frame parts 50, 51, 52, 53 in Figs. 15 to 18 are longer than the expandable frame part 30 and have shapes different from the expandable frame part 30.

In the present embodiment, the recess 21 in which the embryo E is stored in the embryo accommodation portion 20 is disposed closer to the side of the distal end 1B than the other end of each expandable frame part 30. It is considered that the deep side in the uterine cavity 210 has a higher probability of implantation, and in the case of a patient meeting the condition, the above configuration has an effect that the implantation probability can be improved.

In the present embodiment, in the no-load state, when viewed from the direction along the Z axis, an angle α on the side of the distal end 1B (deep side of the uterine cavity 210) among angles formed by the extension direction 43A and the X axis is less than 90° (Fig. 2). That is, in the no-load state, the other end side of each expandable frame part 30 has an inclined portion (third connection portion 43) extending obliquely to the deep side of the uterine cavity 210 (the side of the distal end 1B in the direction along the X axis) from the other end side toward the one end side. The one end side of the third bent portion 33 can also function as the inclined portion. Alternatively, in the no-load state, each expandable frame part 30 has a portion (third connection portion 43, a part of the fourth bent portion 34, etc.) disposed closer to the one end side than the third bent portion 33 and closer to the deep side of the uterine cavity 210 than the terminal on the one end side of the third bent portion 33.

The configuration may have an effect of contributing to prevention of the embryo E being disposed on the fallopian tube side. Also, in the present embodiment, when the pair of width direction outer portions 18 approach each other compared to the no-load state due to force from the uterus etc., force in a direction away from a uterine fundus 230 is likely to be applied to the embryo accommodation portion 20 rather than force in a direction approaching the uterine fundus 230. This can exert an effect of not applying unnecessary force to the uterus as much as possible.

In the embryo transfer device 1 of the present embodiment, the two expandable frame parts 30 position the embryo accommodation portion 20 at the center side in the width direction of the uterine cavity 210 by the expansion and the ring arrangement. By the configuration, the effect that the embryo accommodation portion 20 is not likely to be disposed at the outer side in the width direction of the uterine cavity 210 is obtained, and the effect of prevention or reduction of the embryo E being disposed at the fallopian tube side is obtained.

In each expandable frame part 30 shown in Fig. 2 etc., at least one of the first connection portion 41 which is a portion extending from a terminal on the other end side of the first bent portion 31, a part of the first connection portion 41 and the second bent portion 32, or a part of the second bent portion 32 is an inclined portion extending obliquely toward the entrance side of the uterine cavity 210 (the side of the proximal end 1A in the direction along the X axis) from the one end toward the other end. For this reason, even when there are movements of internal organs such as the uterus and movements of the body equipped with the uterus, there is an effect of reducing a possibility that the embryo transfer device 1 is unintentionally discharged from the uterine cavity 210.

In the present embodiment, the embryo accommodation portion guide 60 is integrally formed with the embryo accommodation portion 20 etc. using the aforementioned mold, and is formed of the same material having rubber-like elasticity as the embryo accommodation portion 20 etc. The present embodiment has a pair of guide portions 61 arranged in the Y direction as the embryo accommodation portion guide 60. A proximal end of each guide portion 61 is fixed to the distal end 1B side of the embryo accommodation portion 20, and a distal end side of each guide portion 61 extends to the side of the distal end 1B and extends in a direction away from the X axis. Thus, each guide portion 61 extends obliquely outward in the Y direction (width direction of the body) from the embryo accommodation portion 20.

Since the configuration is adopted, when the embryo transfer device 1 moves from the tube P into the uterine cavity 210 as described above, even if the embryo transfer device 1 is pressed against the uterine fundus 230 as shown in Fig. 7 etc., the embryo accommodation portion guide 60 comes into contact with the uterine fundus 230 before the embryo accommodation portion 20 does. The configuration is useful for reduction of pressure applied to the uterine fundus 230 as one of its effects.

Also, in the present embodiment, each guide portion 61 is bent or curved in the direction away from the X axis from the proximal end toward the distal end. The configuration is also useful for reduction of pressure applied to the uterine fundus 230 as one of its effects.

Also, in the present embodiment, each guide portion 61 has a tapered portion 61A whose outer diameter gradually decreases from the proximal end toward the distal end (Fig. 2 etc.). In the present embodiment, the tapered portion 61A is formed over substantially the entire length of the guide portion 61. With the configuration, when the embryo transfer device 1 comes out from the tube P as described above in the uterine cavity 210, each guide portion 61 can be smoothly inserted into the uterine cavity 210. The configuration is useful for reducing pressure applied to the uterine cavity 210 as one of its effects.

As shown in Figs. 9 to 12, the connection component 2 of the embryo transfer device kit of the present embodiment has an accommodation section 71A capable of accommodating a part of the embryo transfer device 1. The connection component 2 of the present embodiment has a tube portion 71 extending in the X direction in Fig. 10, and a rotation restricting portion 72 extending from the tube portion 71 in its radial direction thereof.

The accommodation section 71A of the present embodiment is a hollow section of the tube portion 71, and the accommodation section 71A also extends in the X direction (predetermined direction). In the present embodiment, as shown in Fig. 12, the X direction of the embryo transfer device 1 accommodated in the accommodation section 71A is referred to as the X direction of the connection component 2, and the Z direction of the embryo transfer device 1 accommodated in the accommodation section 71A is referred to as the Z direction of the connection component 2.

In the present embodiment, as shown in Fig. 12, a window 73 is formed on a distal end side of the connection component 2, and the window 73 connects the outside of the connection component 2 and the accommodation section 71A in the radial direction of the tube portion 71. The distal end side of the connection component 2 is a side connected to a proximal end portion PD of the tube P when the connection component 2 is connected to the proximal end portion PD as shown in Fig. 14. In the present embodiment, the proximal end portion PD has a hole PH extending in a direction along the central axis of the tube P (longitudinal direction), the distal end side of the connection component 2 is inserted into the hole PH, and the distal end side of the connection component 2 fits into the hole PH. Thereby, the distal end side of the connection component 2 is connected to the proximal end portion PD. An opening side of the hole PH is a tapered hole whose cross-sectional area decreases from the proximal end toward the distal end of the tube P. The configuration is useful for stably moving the embryo E in the embryo accommodation portion 20 to the uterine cavity 210 as one of its effects.

Note that the distal end side of the connection component 2 may be connected to the proximal end portion PD of the tube P using other structures. For example, a fixing component for fixing a proximal end portion of the tube P not provided with the proximal end portion PD and the connection component 2 to each other may be additionally provided, and the proximal end portion of the tube P and the connection component 2 may be connected by the fixing component. As the fixing component, the user can adopt a plastic fixing member gripping the proximal end portion of the tube P and the distal end portion of the tube portion 71 arranged coaxially. The distal end side of the connection component 2 may be connected to the proximal end portion PD of the tube P using other methods and structures.

In the present embodiment, the side of the distal end 1B and the intermediate portion of the embryo transfer device 1 are accommodated in the accommodation section 71A of the connection component 2 before being connected to the proximal end portion PD. The intermediate portion includes a part of the expandable frame part 30 which is the third bent portion 33 etc. In the present embodiment, the user puts the embryo transfer device 1 into the accommodation section 71A in the order of the string 11 and the proximal end portion 10 from the distal end side of the connection component 2. The method is useful for smoothly putting the embryo accommodation portion 20 having the embryo accommodation portion guide 60 into the accommodation section 71A as one of its effects.

The rotation restricting portion 72 restricts the tube portion 71 having the accommodation section 71A so as not to rotate around an axis extending in the X direction. In the case of the present embodiment, the axis is a central axis of the accommodation section 71A. The tube portion 71 of the present embodiment is a tube having a substantially circular cross section, but the cross-sectional shape of the tube portion 71 may be a polygon, an ellipse, etc.

Also, it is possible to adopt a connection component 2' having a shape of Fig. 15. The connection component 2' has, for example, a substantially rectangular parallelepiped shape, and has an accommodation section 71A and a window 73 similar to or the same as those of the connection component 2. In the case of Fig. 15, rotation of the connection component 2' around an axis extending in the X direction is restricted by both end portions in the Y direction of the connection component 2'. That is, both end portions in the Y direction of the connection component 2' function as the rotation restricting portion.

A hole 74 is formed on a distal end side of the connection component 2'. A proximal end portion of the tube P not provided with the proximal end portion PD is inserted into the hole 74, and the proximal end portion fits into the hole 74. Thereby, the distal end side of the connection component 2' is connected to the proximal end portion of the tube P, and the hollow section of the tube P and the accommodation section 71A are arranged coaxially.

When the user puts a part of the embryo transfer device 1 into the accommodation section 71A as shown in Fig. 12, the embryo accommodation portion 20 is disposed at a position corresponding to the window 73. That is, the window 73 is disposed at a position corresponding to an opening of the embryo accommodation portion 20 of the embryo transfer device 1 at least partially accommodated in the accommodation section 71A. Unlike Fig. 12, the entire embryo transfer device 1 may be stored in the accommodation section 71A.

Note that when the user disposes at least a part of the embryo transfer device 1 in the accommodation section 71A as described above, it is sufficient if the user can dispose the opening of the embryo accommodation portion 20 at a position corresponding to the window 73. It can be said that such a window 73 is disposed at a position corresponding to the opening of the embryo accommodation portion 20 of the embryo transfer device 1 at least partially accommodated in the accommodation section 71A.

The user can access the embryo accommodation portion 20 via the window 73. The user places the connection component 2 in which at least a part of the embryo transfer device 1 is disposed in the accommodation section 71A on a predetermined surface such as a bottom surface of a container 110. The container 110 is a petri dish or the like. At this time, in the present embodiment, the user passes the string 11 through the pusher 3 having a pipe shape, and places the connection component 2 on the surface in a state where a pusher distal end which is a distal end of the pusher 3 is disposed in the vicinity of the proximal end portion 10.

Next, the user injects the embryo E and predetermined liquid into the recess 21 of the embryo accommodation portion 20 using an embryo injector 80 as shown in Fig. 13 via the window 73. The embryo injector 80 is a glass dropper, a syringe, or the like in the present embodiment, but other instruments can also be used. In the present embodiment, the predetermined liquid is a culture solution used for culturing the embryo E, liquid containing a culture solution, high-concentration hyaluronic acid, and a growth factor, etc., but other liquids such as body fluid can also be used. In the present embodiment, when injecting the embryo E and the liquid into the recess 21, a part of the liquid enters a gap between the embryo accommodation portion 20 and the accommodation section 71A. Thereby, movement of the embryo transfer device 1 in the accommodation section 71A becomes smooth. Also, an amount of the liquid injected into the window 73 increases, which can suppress concentration change due to evaporation of the liquid.

After disposing the embryo E in the recess 21 and/or when disposing the embryo E in the recess 21, the user observes the embryo E in the recess 21 using a microscope such as a stereoscopic microscope. At this time, if air bubbles exist in the recess 21, observation of the embryo E may be hindered. For this reason, it is preferable that hydrophilic treatment is applied to the inside of the recess 21. The hydrophilic treatment is applied by, for example, deep ultraviolet light irradiation, but the hydrophilic treatment may be performed by other known methods. The hydrophilic treatment may be performed on the entire embryo accommodation portion 20 or the entire embryo transfer device 1.

Note that it is preferable that an inner peripheral surface of the accommodation section 71A and an inner peripheral surface of the tube P consist of a material having a small friction coefficient with respect to the embryo transfer device 1, such as fluororesin.

Next, the user moves the embryo transfer device 1 in which the embryo E and the liquid have been injected into the recess 21, for example, together with the container to the vicinity of a the patient in a state of being accommodated in the connection component 2. Here, the connection component 2 has the rotation restricting portion 72. The configuration exerts an effect that it is easy to maintain a state in which the recess 21 faces upward, which is useful for efficiently performing stable transfer. More specifically, the rotation restricting portion 72 functions so that the connection components 2, 2' do not rotate, for example, when injecting the embryo E into the embryo accommodation portion 20 and when carrying the connection components 2, 2' in a state where the embryo E is stored. Then, as described above, the user connects the distal end side of the connection component 2 as shown in Fig. 14 to the proximal end portion PD of the tube P whose distal end side is disposed in the uterine cavity 210.

Thus, the configuration in which the connection component 2 can accommodate at least a part of the embryo transfer device 1 and the connection component 2 can be connected to the proximal end side of the tube P whose distal end side is disposed in the uterine cavity 210 is useful for safely disposing the embryo E in the recess 21 into the uterine cavity 210 as one of its effects. Conversely, when inserting the distal end side of the tube P into the uterine cavity 210 in a state where the embryo transfer device 1 is disposed in the tube P, it becomes difficult to insert the stylet 4 to the distal end of the tube P at the time of the insertion, and there is a possibility that insertion work of the tube P becomes difficult due to insufficient strength of the distal end portion of the tube P. Note that depending on the shape of the tube P, the shape of the embryo accommodation portion 20, etc., there may be a case where the embryo E accommodated in the recess 21 in the tube P can be safely disposed in the uterine cavity 210 regardless of a rotational position and orientation of the tube P. In this case, a method of inserting the distal end side of the tube P into the uterine cavity 210 in a state where the embryo transfer device 1 is disposed in the tube P can be adopted.

Next, the user moves the embryo transfer device 1 from the connection component 2 to the tube P using the pusher 3 as described above (Fig. 6), and moves the embryo transfer device 1 in the tube P to the uterine cavity 210 (Fig. 7). Then, the user pulls out the tube P from the uterine cavity 210 and the uterine cervix 220, and indwells the embryo transfer device 1 in the uterine cavity 210.

As an example, the embryo transfer device 1 of the present embodiment maintains a state in which the embryo E can contact or is in contact with the endometrium of a target wall in a direction intersecting or substantially orthogonal to the width direction of the uterine cavity 210, not both walls in the width direction of the body of the uterine cavity 210. For this reason, if the embryo accommodation portion 20 rotates around the X axis etc. after coming out from the distal end of the tube P, a possibility that the embryo E falls off from the recess 21, a possibility that the embryo E is damaged, etc. increase. The embryo transfer device 1 of the present embodiment has the embryo accommodation portion guide 60. As one of its effects, the configuration stabilizes the orientation of the embryo accommodation portion 20 when the embryo accommodation portion 20 comes out from the distal end of the tube P and enters the uterine cavity 210. More specifically, the configuration suppresses or prevents rotation of the embryo accommodation portion 20 around the X axis when the embryo accommodation portion 20 comes out from the distal end of the tube P and enters the uterine cavity 210, and can maintain a state in which the opening 22 of the recess 21 contacts or is close to the target wall.

As one of its effects, the configuration may stabilize the orientation of the embryo accommodation portion 20 as described above when the embryo accommodation portion 20 moves in the uterine cavity 210 by force from the pusher 3, when a portion other than the embryo accommodation portion 20 of the embryo transfer device 1 comes out from the tube P by force from the pusher 3, etc.

In order to achieve the effect, a dimension (dimension in the longitudinal direction) between the proximal end and the distal end of each guide portion 61 is preferably 1 time or more, more preferably 1.5 times or more, and further preferably 2 times or more with respect to a maximum dimension of the cross section of the guide portion 61 orthogonal to the longitudinal direction. Note that the configuration of the present embodiment is not limited to these numerical values as long as the above effects can be achieved.

In each of the above embodiments, the embryo transfer device 1 may have an embryo accommodation portion guide 65 shown in Fig. 20 instead of the embryo accommodation portion guide 60. The embryo accommodation portion guide 65 is also integrally formed with the embryo accommodation portion 20 etc. using the aforementioned mold, and is formed of the same material having rubber-like elasticity as the embryo accommodation portion 20 etc.

The embryo accommodation portion guide 65 has a pair of guide portions 66 arranged in the Y direction. A proximal end of each guide portion 66 is fixed to the distal end 1B side of the embryo accommodation portion 20, and distal ends of the pair of guide portions 66 are connected to each other. That is, the embryo accommodation portion guide 65 has a loop shape, or the embryo accommodation portion guide 65 has a loop shape together with a part of the embryo accommodation portion 20.

Each guide portion 66 has a first portion 66A extending from its proximal end to the side of the distal end 1B and extending in a direction away from the X axis. Thus, the first portion 66A of each guide portion 66 extends obliquely outward in the Y direction (width direction of the body) from the embryo accommodation portion 20. Also, each guide portion 66 has a second portion 66B extending from a distal end of the first portion 66A to the side of the distal end 1B and extending in a direction approaching the X axis. The second portions 66B of the pair of guide portions 66 are connected to each other. With these configurations, the embryo accommodation portion guide 65 can exert a similar or the same effects as the embryo accommodation portion guide 60.

In the present embodiment, the embryo accommodation portion guide 65 has a cross-sectional shape shown in Fig. 21 over the entire longitudinal direction thereof. As the cross-sectional shape of the embryo accommodation portion guide 65, an isosceles triangle, a shape close to an isosceles triangle, etc. can be adopted. In the example of Fig. 21, the cross section of the embryo accommodation portion guide 65 has a top portion 65A projecting toward the outer side in the radial direction of the loop shape, and has two sides 65B of substantially straight lines respectively extending obliquely from one side and the other side in the Z direction toward the top portion 65A. With the cross-sectional shape, when the embryo transfer device 1 comes out from the tube P as described above in the uterine cavity 210, the embryo accommodation portion guide 65 can be smoothly inserted into the uterine cavity 210. The configuration is useful for reducing pressure applied to the uterine cavity 210 as one of its effects.

Hereinafter, an embryo transfer device kit according to a second embodiment will be described below with reference to Figs. 22 to 24. The embryo transfer device kit of the second embodiment includes an embryo transfer device 1' shown in Figs. 22 to 24, a connection component 2 similar to or the same as that of the first embodiment, a tube P, a pusher 3 such as a push rod, and a stylet 4. In the second embodiment, the description regarding the configuration similar to or the same as that of the first embodiment is omitted, and the reference signs and names of the similar or the same configurations are used in the second embodiment as necessary.

The embryo transfer device 1' of the second embodiment includes an embryo accommodation portion 20 similar to or the same as that of the first embodiment, and is inserted into the uterine cavity 210 by using the tube P and is indwelt therein, similarly to the first embodiment. The embryo transfer device 1' also has a proximal end 1A and a distal end 1B, and includes a proximal end portion 10 forming at least a part of the proximal end 1A, and an embryo accommodation portion 20 provided on the side of the distal end 1B and accommodating an embryo E. Also in the second embodiment, the X axis passes through the proximal end portion 10 and the embryo accommodation portion 20, and the Z axis is orthogonal to the X axis. Also in the second embodiment, similarly to the first embodiment, the Z axis extends in a direction in which the embryo accommodation portion 20 opens. Furthermore, also in the second embodiment, the Y axis is orthogonal to the X axis and the Z axis.

Also in the second embodiment, an embryo accommodation portion guide 60 similar to or the same as that of the first embodiment extends from the embryo accommodation portion 20. The embryo transfer device 1' of the second embodiment includes a rod-shaped member 54 extending in the X direction instead of the pair of expandable frame parts 30 of the first embodiment. The proximal end of the rod-shaped member 54 is fixed to the proximal end portion 10, and the distal end of the rod-shaped member 54 is fixed to the proximal end portion of the embryo accommodation portion 20.

Also in the second embodiment, similarly to the first embodiment, the embryo accommodation portion guide 60, the embryo accommodation portion 20, the rod-shaped member 54, and the proximal end portion 10 are integrally molded using a mold with the same rubber material as in the first embodiment. Note that in the second embodiment, there may be cases where the embryo accommodation portion guide 60 and the embryo accommodation portion 20 are integrally molded, and the embryo accommodation portion 20 is fixed to the rod-shaped member 54, which is molded as a separate body, with an adhesive or the like. Similarly, in the first embodiment, there may be cases where the embryo accommodation portion 20 is fixed to the other ends of the expandable frame parts 30, which are molded as separate bodies, with an adhesive or the like.

Also in the second embodiment, similarly to the first embodiment, the embryo transfer device 1' is disposed in the accommodation section 71A of the connection component 2, and in that state, the embryo E is disposed in the recess 21. Further, the user connects the connection component 2 to the proximal end portion PD of the tube P, and moves the embryo transfer device 1' in the connection component 2 to the uterine cavity 210 via the tube P by the pusher 3. At this time, the embryo accommodation portion guide 60 expands in the Y direction within the uterine cavity 210.

In the embryo transfer device 1' of the second embodiment, within the uterine cavity 210, the rod-shaped member 54 instead of the expandable frame parts 30 suppresses movement of the indwelled embryo accommodation portion 20, and the embryo accommodation portion guide 60 also suppresses movement of the indwelled embryo accommodation portion 20, similarly to the first embodiment.

Note that, similarly to the first embodiment, it is also possible to provide an embryo accommodation portion guide 65 instead of the embryo accommodation portion guide 60 in the embryo transfer device 1' of the second embodiment.

Hereinafter, an embryo transfer device kit according to a third embodiment will be described below with reference to Fig. 25. The embryo transfer device kit of the third embodiment includes an embryo transfer device 1'' shown in Fig. 25, a connection component 2 similar to or the same as that of the first embodiment, a tube P, a pusher 3 such as a push rod, and a stylet 4. In the third embodiment, the description regarding the configuration similar to or the same as that of the first embodiment is omitted, and the reference signs and names of the similar or the same configurations are used in the third embodiment as necessary.

The embryo transfer device 1" of the third embodiment includes an embryo accommodation portion 20 similar to or the same as that of the first embodiment, and is inserted into the uterine cavity 210 by using the tube P and is indwelt therein, similarly to the first embodiment. The embryo transfer device 1" also has a proximal end 1A and a distal end 1B. Further, the embryo transfer device 1" has an embryo accommodation portion 20 forming at least a part of the proximal end 1A, and an embryo accommodation portion guide 67 similar to the embryo accommodation portion guide 65 of the first embodiment is provided on the side of the distal end 1B with respect to the embryo accommodation portion 20. In one example, the end of the string 11 is fixed to the embryo accommodation portion 20 of the embryo transfer device 1".

The embryo accommodation portion guide 67 has a pair of guide parts 68 arranged in the Y direction. The proximal end of each guide part 68 is fixed to the side of the distal end 1B of the embryo accommodation portion 20, and the distal ends of the pair of guide parts 68 are connected to each other. That is, the embryo accommodation portion guide 67 has a loop shape similarly to the embryo accommodation portion guide 65, or the embryo accommodation portion guide 67 has a loop shape together with a part of the embryo accommodation portion 20.

Each guide part 68 has a first portion 68A extending from its proximal end in a direction away from the X axis. Thus, the first portion 68A of each guide part 68 extends obliquely outward from the embryo accommodation portion 20 in the Y direction (the width direction of the body). Further, a part 68C of the first portion 68A extends toward the side of the distal end 1B and in a direction away from the X axis, from the proximal end toward the distal end of the guide part 68. Further, each guide part 68 has a second portion 68B extending from the distal end of the first portion 68A toward the side of the distal end 1B and in a direction approaching the X axis. The second portions 68B of the pair of guide parts 68 are connected to each other. With these configurations, the embryo accommodation portion guide 67 can exhibit effects similar to or the same as those of the embryo accommodation portion guides 60 and 65.

As shown in Fig. 25, there are cases where widthwise outer sections 18' of each guide part 68 come into contact with both walls in the width direction of the body in the uterine cavity 210, similarly to the widthwise outer sections 18 of the first embodiment. When each guide part 68 is configured in this manner, each guide part 68 more effectively suppresses movement of the indwelled embryo accommodation portion 20 within the uterine cavity 210. In this case, the pair of guide parts 68 can function as the pair of expandable frame parts 30 of the first embodiment.

Also in the third embodiment, similarly to the first embodiment, the embryo transfer device 1'' is disposed in the accommodation section 71A of the connection component 2, and in that state, the embryo E is disposed in the recess 21. Further, the user connects the connection component 2 to the proximal end portion PD of the tube P, and moves the embryo transfer device 1" in the connection component 2 to the uterine cavity 210 via the tube P by the pusher 3. At this time, the embryo accommodation portion guide 67 expands in the Y direction within the uterine cavity 210.

Note that there may be cases where the embryo transfer device 1" further includes the rod-shaped member 54 of the second embodiment connected to the proximal end portion of the embryo accommodation portion 20. In this case, the end of the string 11 is fixed to the proximal end side of the rod-shaped member 54. Further, there may be cases where the embryo transfer device 1" further includes the pair of expandable frame parts 30 of the first embodiment in which the other ends referred to in the first embodiment are fixed to the proximal end portion of the embryo accommodation portion 20. In this case, the proximal end portion 10 is fixed to the one ends referred to in the first embodiment of the pair of expandable frame parts 30, and one end of the string 11 is fixed to the proximal end side of the proximal end portion 10. The other end of the string 11 may be in a ring shape or the like to facilitate removal of the embryo transfer device 1.

In each of the above embodiments, a configuration in which the embryo accommodation portion guide is not provided may be adopted. For example, as shown in Figs. 23 and 24 of the second embodiment, it is possible to make the cross-sectional shape of the proximal end portion of the embryo accommodation portion 20 a substantially polygonal shape such as a substantially hexagonal shape, and to make a part or all of the inner peripheral surface of the tube P a cross-sectional shape complementary to the cross-sectional shape of the proximal end portion of the embryo accommodation portion 20. In the case of Fig. 24, in the cross section of the proximal end portion of the embryo accommodation portion 20, two convex portions (protruding portions) are formed at the positions of the two upper vertices, and two concave portions (grooves) are formed at corresponding positions on the inner peripheral surface of the tube P. In Fig. 24, the two convex portions and the two concave portions are configured to engage with each other around the X axis. This configuration, as one of its effects, suppresses or prevents rotation of the embryo accommodation portion 20 after coming out from the distal end of the tube P.

In Fig. 24, not only the proximal end portion of the embryo accommodation portion 20 but also the cross-sectional shape of the rod-shaped member 54 and/or the cross-sectional shape of the proximal end portion 10 may be similar to or the same as that of the proximal end portion of the embryo accommodation portion 20. This configuration, as one of its effects, more effectively suppresses or prevents rotation of the embryo accommodation portion 20 after coming out from the distal end of the tube P.

Further, a protruding portion may be provided on at least one of the embryo accommodation portion 20, the rod-shaped member 54, or the proximal end portion 10, and a groove that engages with the protruding portion around the X axis may be formed on the inner peripheral surface of the tube P. This configuration also, as one of its effects, suppresses or prevents rotation of the embryo accommodation portion 20 after coming out from the distal end of the tube P. Note that the protruding portion may be provided on the inner peripheral surface of the tube P, and the groove may be provided on at least one of the embryo accommodation portion 20, the rod-shaped member 54, or the proximal end portion 10. The configuration in which the convex portion and the concave portion engage with each other around the X axis, or the configuration in which the protruding portion and the groove engage with each other around the X axis, is also adopted as appropriate in the first embodiment and the third embodiment.

Furthermore, the cross-sectional shape of the inner peripheral surface of the tube P is preferably substantially elliptical or substantially polygonal. As described in each of the above embodiments, the pair of expandable frame parts 30, 50, 51, 52, 53 and the pair of guide parts 61, 66, 68 of the embryo accommodation portion guides 60, 65, 67 are in a state of being close to each other when inside the tube P, and at this time, a restoring force in a direction opening in the Y direction is generated. Therefore, if the cross-sectional shape of the inner peripheral surface of the tube P is elliptical or polygonal, the pair of expandable frame parts 30, 50, 51, 52, 53 and the pair of guide parts 61, 66, 68 are difficult to rotate around the X axis within the tube P due to the restoring force. That is, the fact that the cross-sectional shape of the inner peripheral surface of the tube P is elliptical or polygonal, as one of its effects, suppresses or prevents rotation of the embryo accommodation portion 20 after coming out from the distal end of the tube P. Thus, the suppression of rotation around the X axis in the tube P of the embryo transfer devices 1, 1', 1'', the expandable frame parts 30, 50, 51, 52, 53, the guide parts 61, 66, 68, etc. can be typically realized by the fact that the cross section perpendicular to the X axis of the inner peripheral surface of the tube P is not perfectly circular as described above. In this case, as described above, even if the cross section perpendicular to the X axis of many parts of the embryo transfer devices 1, 1', 1'' is circular, the rotation suppression can be realized. The state in which the cross section of the inner peripheral surface of the tube P is not perfectly circular is naturally also realized by providing a single or a plurality of protrusions or grooves extending in the X-axis direction on the inner peripheral surface having a circular cross section. When providing a plurality of protrusions or a plurality of grooves, the height of each protrusion or the depth of each groove is 3 µm or more, preferably 8 µm or more, and more preferably 10 µm or more. Even with such small protrusions or grooves, the rotation suppression is sufficiently achieved, and this contributes to securing space in the tube P, reducing the diameter of the tube P, etc., as one of its effects.

In each of the above embodiments, a configuration in which the embryo accommodation portion guides 60, 65, 67 are not provided may be adopted. Even in this case, the rotation of the embryo accommodation portion 20 can be suppressed or prevented by the above-described configuration or other configurations.

Further, a rotation restricting structure may be provided on the distal end side of the connection component 2, 2'. The rotation restricting structure restricts rotation of the connection component 2, 2' relative to the tube P around the axis extending in the X direction. As the rotation restricting structure, for example, a convex portion (protruding portion) is formed on the outer peripheral surface of the distal end side of the tube part 71. In this case, a concave portion (groove) is formed at a position corresponding to the convex portion on the inner peripheral surface of the hole PH of the tube P. As the rotation restricting structure, the distal end side of the tube part 71 may be formed in a polygonal cross section. In this case, the hole PH is formed so that the distal end side of the tube part 71 engages around the axis. The rotation restricting structure is useful for realizing stable transfer of the embryo E as one of its effects.

Note that there may be cases where the embryo transfer device 1 has three or more expandable frame parts. For example, in the embodiment of Fig. 2, one expandable frame part 30 in the Y direction may consist of two expandable frame parts arranged to overlap in the Z direction, and the other expandable frame part 30 in the Y direction may consist of two expandable frame parts arranged to overlap in the Z direction. In this case, the embryo transfer device 1 will have four expandable frame parts.

Further, another member such as a thin rubber frame, string, or plastic frame may be disposed between the pair of expandable frame parts of Fig. 2 etc. of the embryo transfer device 1, and one end of the member may be connected to the proximal end portion 10, and the other end of the member may be connected to the embryo accommodation portion 20. Thus, in each of the above embodiments, additional members, structures, etc. not explicitly stated in the above description may be added as appropriate.

It is also possible to provide the embryo accommodation portion 20 at the distal end 1B of the embryo transfer device 1" shown in Fig. 25. In this case, two embryo accommodation portions will be provided in the embryo transfer device 1". Alternatively, it is also conceivable to provide the embryo accommodation portion 20 on each of the pair of expandable frame parts 30 of the embryo transfer device 1. In this case, a plurality of embryo accommodation portions will be provided in the embryo transfer device 1. It is also possible not to connect the distal ends of the two guide parts 68 of the embryo transfer device 1". Even in this case, the guide part 68 serves as resistance to movement of the embryo accommodation portion 20 within the uterine cavity 210.

Further, as shown in Fig. 26, a flat surface part (curvature reducing part) 73A may be provided around the window 73 in the connection component 2. There may be a portion around the window 73 where the flat surface part 73A is not provided. In the case of Fig. 26, the flat surface part 73A is provided over the entire X direction of the window 73 on both sides in the Y direction with respect to the window 73, and the flat surface part 73A is not provided in other parts. When the flat surface part 73A is present, as shown in Fig. 26, when the injected liquid becomes a convex shape due to surface tension at the window 73, the curvature of the central liquid surface WS at the central part of the window 73 is reduced. This configuration, as one of its effects, is useful for accurately performing observation with a microscope or the like in the recess 21 in a state where the liquid is injected. Note that an extended part 71B extending in the Y direction from the tube part 71 may be provided as the curvature reducing part as shown in Fig. 27.

In the first embodiment, the embryo transfer device 1, the connection component 2, the tube P, the pusher 3, and the stylet 4 may be sold in a state of being accommodated in the same package, or some of these may be accommodated in the same package and sold. Further, in the first embodiment, there may be cases where the embryo transfer device kit does not have the tube P, the pusher 3, and the stylet 4. Even in this case, the user can indwell the embryo transfer device 1 accommodating the embryo E in the embryo accommodation portion 20 in the uterine cavity 210 as described above, using other tubes, push rods, stylets, etc. This modification of the first embodiment can also be applied to the embryo transfer device kits of the second embodiment and the third embodiment.

As the string 11, it is possible to use a synthetic resin multifilament string, a monofilament string, or other known strings. On the other hand, as shown in Fig. 28, a string 11' may be integrally formed with the embryo transfer device 1 using the mold. In this case, the string 11' is also formed of a material having the same rubber elasticity as the embryo transfer device 1. In this case, it is also possible to form a coating layer 12 made of a synthetic resin or a material having rubber elasticity around the string 11'. The material having rubber elasticity of the coating layer 12 preferably has a rubber hardness 5 points or more higher than the material of the embryo transfer device 1, and more preferably 10 points or more higher. The coating layer 12, as one of its effects, prevents or suppresses elongation of the string 11', which can improve workability when pulling out the embryo transfer device 1 from the uterine cavity 210.

The pair of guide parts 61, 66, 68 of the embryo accommodation portion guides 60, 65, 67 become a state of being close to each other in the connection component 2 and the tube P, and when released from the tube P, they separate from each other in the Y direction, whereby the pair of guide parts 61, 66, 68 expand. It can also be said that when released from the tube P, the pair of guide parts 61, 66, 68 expand in a direction away from the center axis line CL on the distal end side of the tube P. Such a configuration of the pair of guide parts 61, 66, 68 is useful for reducing damage to the uterine cervix 220, the uterine cavity 210, etc. at the time of insertion/withdrawal of the device and improving the success rate of implantation, as one of its effects. Note that one guide part 61, 66, 68 in the Y direction may consist of two guide parts arranged to overlap in the Z direction, and the other guide part 61, 66, 68 in the Y direction may consist of two guide parts arranged to overlap in the Z direction. In this case, the embryo transfer devices 1, 1', 1'' will have four expandable frame parts.

Here, in the intermediate portion in the X direction of the recess 21 of the embryo accommodation portion 20 shown in Fig. 13, it is preferable that the thickness dimension of the wall 21A in the vicinity of the opening 22 is thin. In this embodiment, the thickness dimension of the wall 21A is 0.15 mm or less. The thickness dimension of the wall 21A is preferably 0.2 mm or less, more preferably 0.15 mm or less, and further preferably 0.12 mm or less. In this embodiment, the height dimension of the wall 21A in the direction along the Z axis is 0.4 mm or more. The height dimension is preferably 0.3 mm or more. It is preferable that 0.2 mm or more, more preferably 0.3 mm or more in the vicinity of the opening 22 of the wall 21A in the direction along the Z axis has the above thickness dimension. Note that the effects described later can be achieved even with dimensions other than the above. Further, even if the thickness dimensions of the walls other than the intermediate portion in the X direction of the recess 21 or the walls 21B, 21C (Fig. 29) at one end and the other end in the X direction of the recess 21 are not the above dimensions, the effects described later can be achieved.

The wall 21A in the vicinity of the opening 22 of the recess 21 of this embodiment elastically deforms toward the inside of the recess 21 within the connection component 2, as shown by the double-dotted chain line in Fig. 13. In this embodiment, the wall 21A hardly elastically deforms in the range where the window 73 of the connection component 2 is present, and the wall 21A elastically deforms as shown by the double-dotted chain line in Fig. 13 in the range where the window 73 of the connection component 2 is present. The wall 21A in the vicinity of the opening 22 similarly elastically deforms in the tube P. When the embryo accommodation portion 20 is released from the tube P, the wall 21A returns or tries to return to the shape before elastic deformation due to the restoring force of elastic deformation.

This configuration, as one of its effects, is useful for increasing the volume in the recess 21. The embryo accommodation portion 20 is formed to pass through the thin tube P, and accordingly, the volume of the recess 21 is also limited. Further, the concentration of the liquid accommodated in the recess 21 together with the embryo E changes due to evaporation or the like, and if the volume of the recess 21 is small, the concentration change of the liquid is fast. Since the concentration of the liquid may affect the implantation probability, the above configuration capable of increasing the volume in the recess 21 is useful.

Note that the inner diameter of the connection component 2 does not need to be constant. For example, it is possible to adopt a configuration in which the diameter of the inner peripheral surface of the tube part 71 of the connection component 2 gradually decreases toward the distal end of the connection component 2, and it is also possible to adopt a configuration in which the cross-sectional shape of the inner peripheral surface of the connection component 2 changes.

The wall 21A that softly elastically deforms toward the inside of the recess 21 within the connection component 2 and the tube P in this way tends to elastically deform also when the embryo accommodation portion 20 comes out from the distal end of the tube P and enters the uterine cavity 210, when moving within the uterine cavity 210, etc. Although this is not preferable for stabilizing the orientation of the embryo accommodation portion 20 within the uterine cavity 210, in this embodiment, the orientation of the embryo accommodation portion 20 is stabilized by the embryo accommodation portion guides 60, 65, 67. That is, this embodiment, as one of its effects, can stabilize the orientation of the embryo accommodation portion 20 within the uterine cavity 210 while suppressing the concentration change of the liquid in the recess 21.

Fig. 29 shows a modification in the XXIX-XXIX line cross section of Fig. 2. As shown in Fig. 29, the inner surfaces of the wall 21B at one end and the wall 21C at the other end in the X direction in the recess 21 may be inclined so as to be away from each other from the bottom of the recess 21 toward the opening. In this embodiment, the inner surfaces of the walls 21B, 21C are inclined by 10° or more with respect to the Z axis, and more preferably inclined by 15° or more. This configuration, as one of its effects, can realize that the embryo E moves smoothly to the target wall in a state where the opening 22 of the recess 21 is in contact with or close to the target wall of the uterine cavity 210. Also, this configuration, as one of its effects, is useful for making it difficult for air bubbles to stay in the recess 21. In order to make it difficult for air bubbles to stay in the recess 21, it is preferable that a corner R is formed at the corner in the recess 21. It can also be said that the possibility that the embryo E stays in the corner in the recess 21 when the inner side wall is parallel to the Z axis is reduced. Note that the inner surfaces of the walls 21A on both sides in the Y direction in the cross section shown in Fig. 13 may be similarly inclined.

Further, as shown in Fig. 29, a single or a plurality of perfusion holes 21D communicating from the outside of the embryo accommodation portion 20 to the inside of the recess 21 may be provided in the embryo accommodation portion 20. This configuration, as one of its effects, assists the replacement of the liquid inside the recess 21 with the liquid outside the embryo accommodation portion 20 in the uterine cavity 210. In this embodiment, the inner diameter of the perfusion hole 21D is smaller than the outer diameter of the embryo E, and in one example, the inner diameter of the hole 21D is 0.1 mm or less.

Further, it is preferable that a mark M shown in Fig. 7 is provided on the proximal end side of the pusher 3. A scale may be provided instead of the mark M. The mark M or the scale is a mark for the user to recognize the insertion amount of the pusher 3 into the tube P. In the example shown in Fig. 7, when the pusher 3 is inserted into the tube P to a position necessary to push out the entire embryo transfer device 1 from the tube P, the mark M is configured to match the proximal end of the tube P or the connection component 2. That is, the mark M or the scale indicates that the pusher 3 has been inserted to a position necessary to push out the entire embryo transfer device 1 from the tube P.

Further, as shown in Figs. 30 and 31, it is also possible to make the rod-shaped member 54 thin in the second embodiment so that the rod-shaped member 54 is inserted into the pipeshaped pusher 3. In this case, as shown in Figs. 30 and 31, an engaging portion 3A, which is a notch, may be provided at the distal end of the pusher 3, and an engaged portion 25 engaging with the engaging portion 3A in the circumferential direction of the pusher 3 may be provided at the distal end portion of the rod-shaped member 54 or the embryo accommodation portion 20. This configuration, as part of its effects, can prevent or suppress the embryo accommodation portion 20 from rotating around the central axis line of the tube P when the user pushes the embryo transfer device 1 in the connection component 2 and the tube P with the pusher 3.

Note that a protrusion may be provided at the distal end of the pusher 3, and a recess, a groove, or the like corresponding to the protrusion may be provided at the distal end portion of the embryo accommodation portion 20 or the rod-shaped member 54. Further, the engaging portion 3A may be a groove or the like on the inner peripheral surface of the pusher 3. It is also possible to adopt another configuration in which the engaging portion 3A and the engaged portion 25 engage with each other in the circumferential direction. Further, this configuration can be applied to the third embodiment, and can also be applied to the first embodiment in which the expandable frame parts 30 and the proximal end portion 10 are made thin.

In the above embodiment, it is described that a hydrophilic treatment is performed in the recess 21, but it is also conceivable to perform a hydrophobic treatment in the recess 21. The hydrophobic treatment can be performed by a known method such as fluororesin coating or surface film formation by CVD. When the hydrophobic treatment is performed in the recess 21, the embryo E easily comes out of the recess 21. The structure in which the inner surfaces of the wall 21B and the wall 21C are inclined so as to separate from each other from the bottom of the recess 21 toward the opening is also useful for the embryo E to easily come out of the recess 21. The discharge means for facilitating the embryo E to come out of the recess 21 in this way is useful for improving the implantation probability. For example, when the user can observe the embryo E in the recess 21 with a microscope without any problem, or when it is not necessary to observe the embryo E in the recess 21, the discharge means improves the implantation probability, so it is extremely useful in the technical field. It is naturally possible to perform Parylene coating, which is often used in medical devices, in the recess 21 as the treatment. By coating the recess 21 made of a material having rubber elasticity such as silicon rubber with Parylene by Parylene coating, effects such as improvement in biocompatibility of the recess 21 (higher survival rate of embryo) and facilitating rolling of the embryo E in the recess 21 are expected. When parts other than the recess 21 of the embryo transfer device are coated by Parylene coating, an effect that the slidability of the embryo transfer device in the tube P can be improved is obtained.

Further, a perfusion hole similar to or the same as the hole 21D may be provided in any one of the walls 21A, 21B, and 21C of the recess 21. Further, when the embryo accommodation portion 20 is disposed in the tube P or the accommodation section 71A, any one of the walls 21A, 21B, and 21C may elastically deform so as to be folded toward the inside of the recess 21. In this case, when the embryo accommodation portion 20 is released from the tube P, the folded portion of any of the walls 21A, 21B, and 21C deforms in the restoring direction due to the reaction force of elastic deformation.

In each of the above embodiments, after injecting the embryo E and the liquid into the recess 21, the user may place a biodegradable film 90 at the opening of the recess 21 using an instrument such as tweezers so as to close the opening of the recess 21 as shown in Fig. 32. In this embodiment, placing the film 90 so as to close the opening of the recess 21 means placing the film 90 so as to close 50% or more of the opening area. Preferably, the film 90 closes 70% or more of the opening area, and more preferably, the film 90 closes 80% or more of the opening area. The film 90 may be disposed such that the edge of the film 90 adheres to any or all of the walls 21A, 21B, and 21C of the recess 21.

Alternatively, as shown in Fig. 33, before the embryo E and the liquid are injected into the recess 21, the film 90 may be disposed at the opening so as to close the opening of the recess 21. In this case, the edge of the film 90 is fixed to any or all of the walls 21A, 21B, and 21C of the recess 21 by a biodegradable pressure-sensitive adhesive or adhesive. In this case, when injecting the embryo E and the liquid into the recess 21 using the embryo injector 80 as in each of the above embodiments, a hole can be made in the film 90 with the tip of the embryo injector 80, and the embryo E and the liquid can be injected into the recess 21 from the hole.

As the film 90, the pressure-sensitive adhesive, or the adhesive, a known biodegradable material can be used. The film 90, the pressure-sensitive adhesive, and the adhesive are decomposed or become a dissolved state by the liquid or body fluid, preferably in several minutes, more than ten minutes, several tens of minutes, or several hours. The dissolved state is a state in which the opening of the recess 21 is not substantially closed by the film 90 and the embryo E can come out of the recess 21.

In each of the above configurations, as one of its effects, the film 90 prevents the embryo E in the recess 21 from falling off before the embryo accommodation portion 20 comes out of the tube P and is disposed in the uterine cavity 210. Further, in each of the above configurations, as one of its effects, evaporation of the liquid in the recess 21 is suppressed by the film 90.

Furthermore, another opening which opens in a direction opposite to the opening 22 in the Z direction may be provided at a position corresponding to the bottom surface of the recess 21 in the embryo accommodation portion 20. For example, the other opening is provided on the entire bottom surface of the recess 21. In this case, for example, the edge of the film 90 is fixed to the other opening, and the film 90 is disposed so as to close the other opening. For example, the film 90 completely closes the other opening, or closes 90% or more of the opening area. After the embryo transfer device 1 is disposed in the uterine cavity 210, when the film 90 decomposes or becomes a dissolved state and the other opening opens, there may be cases where the embryo E implants on the uterine inner wall on the side of the other opening.

In each of the above embodiments, an inclined part 71C extending obliquely upward may be formed at one of the one end and the other end of the accommodation section 71A of the connection component 2. When the connection component 2 is viewed from the Y direction as in Fig. 34, the direction in which the central axis line CL2 of the inclined part 71C extends is inclined by 30° with respect to the direction in which the central axis line CL1 of the central part of the accommodation section 71A extends, but the inclination may be 3° or more, and preferably 5° or more. When injecting the liquid and the embryo E into the recess 21 of the embryo accommodation portion 20 in the accommodation section 71A, a part of the liquid that did not enter the recess 21 enters the gap between the embryo accommodation portion 20 and the inside of the accommodation section 71A as shown in Figs. 26, 27, etc. The configuration of the inclined part 71C, as one of its effects, prevents the liquid entering the gap from coming out from the end portion of the accommodation section 71A, which is useful for suppressing concentration change due to evaporation of the liquid. The above idea of providing the inclined part 71C also includes forming the entire accommodation section 71A so as to be curved downward. Even in this case, the inclined part 71C is formed at at least one of the one end and the other end of the accommodation section 71A, or the inclination of the central axis line CL2 of the inclined part 71C with respect to the X axis is 3° or more as described above. Even if the angle is less than 3°, if the effect of preventing the liquid from coming out from the end portion of the accommodation section 71A is achieved as described above, the configuration of the inclined part 71C is useful.

Hereinafter, an embryo transfer device kit according to a fourth embodiment will be described below with reference to Figs. 35 to 44. The embryo transfer device 5 of the fourth embodiment is a modification of the embryo transfer device 1' of the second embodiment as shown in Figs. 35 to 37. In the embryo transfer device 5, the description regarding the configuration similar to or the same as that of the second embodiment is omitted, and the reference signs and names of the similar configuration are used in the fourth embodiment as necessary.

The configuration of the embryo accommodation portion guide 60 on the side of the distal end 1B of the embryo transfer device 5 is similar to or the same as that of the second embodiment, and the side of the proximal end 1A of the embryo transfer device 5, the rod-shaped member 54, and the like are different from those of the second embodiment.

A plurality of expandable parts 13 are provided at the proximal end portion 10 of the embryo transfer device 5. The proximal end of each expandable part 13 is fixed to the proximal end portion 10, and the side of the other end of each expandable part 13 extends in a direction away from the X axis. Similarly to the expandable frame parts 30 and the guide parts 61, the plurality of expandable parts 13 are in a state of being close to each other when entering the tube P, and expand in a direction away from the X axis when coming out from the tube P into the uterine cavity 210. In the fourth embodiment, the plurality of expandable parts 13 expand in the Y direction.

A hole 54A extending in the X direction is formed inside the rod-shaped member 54 and the proximal end portion 10. As shown in Fig. 37, a wall 54B is provided between the hole 54A and the recess 21 of the embryo accommodation portion 20. That is, the hole 54A extends from the proximal end 1A of the embryo transfer device 5 to the wall 54B.

A through-hole 54C connecting the hole 54A and the recess 21 is formed in the wall 54B. The hole diameter of the through-hole 54C is smaller than the hole diameter of the hole 54A, and is preferably 1/2 or less of the hole diameter of the hole 54A. The through-hole 54C may be a through-hole of another shape such as a cross shape. Further, in one example, the hole diameter of the through-hole 54C is equal to the outer diameter of the embryo E, or is equal to or less than the outer diameter of the embryo E.

In the embryo transfer device kit according to the fourth embodiment, a pusher 6 is used instead of the pusher 3. The pusher 3 pushes only the proximal end portion 10, but in the fourth embodiment, the pusher 6 may also push the embryo accommodation portion 20 and its vicinity. The pusher 6 also has a thin pipe shape made of plastic, metal, etc., and the distal end portion 6A of the pusher 6 is thinner than its proximal end side as shown in Figs. 38 to 42. The outer diameter of the distal end portion 6A is preferably 1.2 mm or less, more preferably 1 mm or less, but the outer diameter of the distal end portion 6A may exceed 1.2 mm.

As shown in Fig. 42, a discharge device 7 such as a micropipette or a microsyringe is attached to the proximal end of the pusher 6. The discharge device 7 can suck gas and/or liquid within the inside of the proximal end side of the pusher 6, and can supply gas and/or liquid to the inside of the proximal end side of the pusher 6. It is also possible to use a discharge device 7 such as a micropipette whose cylinder is moved by a motor.

As shown in Figs. 38 to 41 etc., the distal end of the distal end portion 6A opens not only in the X direction but also in a direction crossing the X direction (Z direction and/or Y direction). For this purpose, an X-direction opening 6B and a crossing-direction opening 6C are formed at the distal end of the distal end portion 6A. Note that there may be cases where only the X-direction opening 6B is provided at the distal end of the distal end portion 6A.

In the first to third embodiments, the user injected the embryo E and the liquid into the recess 21 of the embryo accommodation portion 20 disposed in the accommodation section 71A using the embryo injector 80 or the like. In the fourth embodiment, the user puts the embryo E and the liquid into the pusher 6. For this purpose, the user inserts the distal end portion 6A into a container containing the embryo E and the liquid, and in this state, sucks gas and/or liquid inside the proximal end side of the pusher 6 by the discharge device 7. Thereby, the embryo E and the liquid are disposed inside the pusher 6 as shown in Fig. 38. Typically, more liquid than the state shown in Fig. 38 is disposed inside the pusher 6.

Next, the user inserts the distal end portion 6A into the hole 54A from the side of the proximal end 1A of the embryo transfer device 5. When a groove or a notch extending in the X direction is formed in the rod-shaped member 54 instead of the hole 54A, the user puts the distal end portion 6A into the groove or the notch. In this embodiment, this insertion is performed in a state where the embryo transfer device 5 is disposed in the accommodation section 71A (Fig. 40). Thus, preferably, the insertion operation is performed at a position that does not affect the patient's body. Note that the insertion may be performed before the embryo transfer device 5 is disposed in the accommodation section 71A.

At the time of the insertion of the distal end portion 6A into the hole 54A, the distal end side of the distal end portion 6A is inserted through the through-hole 54C, and the distal end of the distal end portion 6A is disposed in the recess 21. Since the wall 54B is formed of the aforementioned material having rubber elasticity similarly to the embryo accommodation portion 20 and the rod-shaped member 54, the distal end side of the distal end portion 6A is inserted through the through-hole 54C by the distal end portion 6A pushing and widening the through-hole 54C. Therefore, the through-hole 54C returns to its original size after the withdrawal of the pusher 6 described later.

Subsequently, the user moves the embryo transfer device 5 to the vicinity of the patient in a state of being accommodated in the connection component 2. Then, the user connects the distal end side of the connection component 2 to the proximal end portion PD of the tube P whose distal end side is disposed in the uterine cavity 210 as described above, and in that state, disposes the embryo transfer device 5 into the uterine cavity 210 via the tube P by the pusher 6 (Fig. 42). The movement, function, etc. of the embryo accommodation portion guide 60 at this time are similar to or the same as those in the first to third embodiments.

Subsequently, in order to dispose the embryo E in the recess 21, the user supplies gas and/or liquid to the inside of the proximal end side of the pusher 6 by the discharge device 7. At this time, preferably, the supply amount of gas and/or liquid per unit time is adjusted to an extremely small amount. Thereby, the embryo E is disposed in the recess 21 as shown in Fig. 41.

Subsequently, as shown in Fig. 41, the user pulls the pusher 6 in the direction of arrow A2 while applying force to the tube P in the direction of arrow A1 as necessary, and pulls out the pusher 6 from the tube P. At this time, as shown in Fig. 42, the plurality of expandable parts 13 released from the tube P expand in the Y direction. The expanding expandable parts 13 prevent or suppress the proximal end 1A of the embryo transfer device 5 from re-entering the tube P, the embryo transfer device 5 from moving to the side of its proximal end 1A, etc.

Then, the user withdraws the tube P from the uterine cavity 210 and the uterine cervix 220, and indwells the embryo transfer device 5 in the uterine cavity 210. Even in this case, the embryo transfer device 5 exhibits the aforementioned effects of the embryo transfer device 1' disposed in the uterine cavity 210, and the embryo accommodation portion guide 60 also exhibits the aforementioned effects. Note that it is also possible to eliminate the bottom wall of the recess 21 of the embryo accommodation portion 20 of the embryo transfer device 5 and make the recess 21 a hole penetrating in the Z direction.

Further, it is also possible to adopt the aforementioned embryo accommodation portion guide 65 instead of the embryo accommodation portion guide 60 as shown in Fig. 43. Further, it is also possible to adopt the aforementioned embryo accommodation portion guide 67 instead of the embryo accommodation portion guide 60. In this case, the proximal end 1A of the embryo transfer device 5 may be the embryo accommodation portion 20 as in Fig. 25.

Further, as shown in Fig. 44, there may be cases where the opening of the distal end of the distal end portion 6A of the pusher 6 is disposed in a range surrounded by the embryo accommodation portion guides 65, 67. In Fig. 44, a through-hole similar to or the same as the through-hole 54C is also provided in the wall 21B of the recess 21. In this case, the user supplies gas and/or liquid to the inside of the proximal end side of the pusher 6 by the discharge device 7 in order to dispose the embryo E in the surrounded range. In this case, the embryo transfer device 5 is indwelt in the uterine cavity 210 in a state where the embryo E is disposed in the surrounded range. This configuration and method may also exhibit effects similar to or the same as the case where the embryo E is disposed in the recess 21. Note that in the case of Fig. 44, it is also possible to use an embryo transfer device 5 without the embryo accommodation portion 20.

When forming the expandable frame part 30 using a mold as described above, a string-shaped member, a band-shaped member, a thread-shaped member, or the like in which the small spheres or the particles are embedded can be disposed at a position corresponding to the expandable frame part 30 of the mold. When insert molding is used in this way, the small spheres or the particles can be reliably disposed in a thin portion such as the expandable frame part 30. Further, by dissolving a base material having rubber elasticity such as TPU (thermoplastic polyurethane elastomer) in a solvent, mixing the small spheres or the particles into the solution, applying the mixed solution to the expandable frame part 30, and drying it, it is also possible to dispose the small spheres or the particles in a thin portion such as the expandable frame part 30. Note that it is also possible to attach a metal marker to, for example, the widthwise outer section 18 of the expandable frame part 30 depending on conditions.

Although embodiments of the present disclosure have been described in detail, the present disclosure is not limited to the individual embodiments described above. These embodiments allow various additions, substitutions, modifications, partial omissions, etc. within the scope not departing from the gist of the invention, or within the scope not departing from the idea and spirit of the present invention derived from the contents described in the claims and their equivalents. For example, in the above-described embodiments, changing the order of each operation, changing the order of each process, omitting or adding a part of operations according to conditions, and omitting or adding a part of processes according to conditions are possible without being limited to the above examples. The same applies when numerical values or mathematical formulas are used in the description of the above embodiments.

### {Reference Signs List}

- 1, 1', 1" :: Embryo transfer device
- 1A :: Proximal end
- 1B :: Distal end
- 2, 2' :: Connection component
- 3 :: Pusher
- 3A :: Engaging portion
- 4 :: Stylet
- 5 :: Embryo transfer device
- 6 :: Pusher
- 7 :: Discharge device
- 10 :: Proximal end portion
- 11,: 11' : String
- 12 :: Coating layer
- 20 :: Embryo accommodation portion
- 21 :: Recess
- 21A, 21B, 21C :: Wall
- 21D :: Hole
- 22 :: Opening
- 25 :: Engaged portion
- 30, 50, 51, 52, 53 :: Expandable frame part
- 54 :: Rod-shaped member
- 54A :: Hole 54A
- 54B :: Wall
- 54C :: Through-hole
- 60, 65, 67 :: Embryo accommodation portion guide
- 61, 66, 68 :: Guide part
- 71 :: Tube part
- 71A :: Accommodation section
- 72 :: Rotation restricting portion
- 73 :: Window
- 73A :: Flat surface part
- E :: Embryo
- P :: Tube
- PD :: Proximal end portion
- PH :: Hole
- CL :: Central axis line

## Claims

1. An embryo transfer device kit comprising:
a connection component connected to a proximal end of a tube having a distal end to be disposed inside a uterus and the proximal end to be disposed outside the uterus; and
an embryo transfer device at least one part of which is accommodated in an accommodation section provided in the connection component so as to extend in a predetermined direction,
wherein the embryo transfer device comprises an embryo accommodation portion having a recess opening in a direction crossing the predetermined direction, and
wherein the embryo accommodation portion is for keeping an embryo, which is disposed in the recess, in a space surrounded by the recess and an endometrium of a uterine cavity existing in an opening direction of the recess, in a state in which the embryo accommodation portion is disposed in the uterine cavity.

2. The embryo transfer device kit according to claim 1,
wherein the connection component has a window at a position corresponding to the embryo accommodation portion of the embryo transfer device in which the at least one part is accommodated in the accommodation section, and
wherein the window enables access to the recess of the embryo accommodation portion from outside of the connection component.

3. The embryo transfer device kit according to claim 1, comprising a pusher for moving the embryo transfer device into an inside of the tube in a state in which the at least one part is accommodated in the connection component connected to the proximal end of the tube, to inside of the tube,
wherein the pusher is configured so that the pusher can push out the embryo transfer device, which is moved into the inside of the tube, from the distal end of the tube.

4. The embryo transfer device kit according to any one of claims 1 to 3, wherein the connection component has a rotation restricting portion that restricts rotation around an axis extending in the predetermined direction when placed on a flat surface.

5. The embryo transfer device kit according to any one of claims 1 to 3, wherein a rotation restricting structure is provided on a distal end side of the connection component, and the rotation restricting structure restricts rotation of the connection component relative to the tube.

6. The embryo transfer device kit according to any one of claims 1 to 3, wherein a hole whose cross-sectional area decreases from the proximal end toward the distal end of the tube is formed on a side of the proximal end of the tube.

7. The embryo transfer device kit according to claim 3,
wherein the pusher has a pusher distal end and a pusher proximal end, and the pusher is inserted into the proximal end of the tube from the pusher distal end, and
wherein the pusher is provided with a mark or a scale capable of allowing a user to recognize a position of the pusher distal end relative to the distal end of the tube.
